# EUROPÄISCHE PATENTSCHRIFT

(11) **EP 4 194 045 B1**
(45) Veröffentlichungstag und Bekanntmachung des Hinweises auf die Patenterteilung: **22.07.2026**
(21) Anmeldenummer: 23153829.9
(22) Anmeldetag: 29.04.2016
(51) Int. Cl.: A61M 60/13, A61M 60/226, A61M 60/237, A61M 60/414, A61M 60/808, A61M 60/81

(54) **ROTOR FÜR EINE FLUIDPUMPE**
ROTOR FOR A FLUID PUMP
ROTOR DE POMPE À FLUIDE

(30) Priorität: 30.04.2015 EP 15166042; 30.04.2015 EP 15166045
(43) Veröffentlichungstag der Anmeldung: 14.06.2023
(62) Teilanmeldung aus: 16719427.3
(73) Patentinhaber: ECP Entwicklungsgesellschaft mbH, 52074 Aachen (DE)
(72) Erfinder: SIESS, Thorsten, 52074 Aachen (DE); SCHECKEL, Mario, 14195 Berlin (DE)
(74) Vertreter: Pfenning, Meinig & Partner mbB

(56) Entgegenhaltungen:
- EP-A1- 2 407 185
- EP-A1- 2 407 187
- US-A1- 2013 177 409
- US-A1- 2014 301 822

## Beschreibung

Die vorliegende Schutzrechtsanmeldung liegt auf dem Gebiet der Mechanik und befasst sich konkret mit Rotoren für Fluidpumpen. Sie ist mit besonderem Vorteil im Bereich der Medizintechnik bei Katheterpumpen einsetzbar.

Im Bereich der Fluidpumpen sind Rotorpumpen bereits in verschiedenen Ausführungsformen als Axialpumpen oder Radialpumpen bekannt. In beiden Fällen wird das zu fördernde Fluid durch eine Rotation eines Rotors und an diesem befestigter Förderelemente beschleunigt, entweder in Axialrichtung oder in Radialrichtung.

Derartige Pumpen können grundsätzlich auch nach dem Stand der Technik bereits komprimiert werden, um sie platzsparend anzuordnen oder zu transportieren. Dies trifft insbesondere auf Katheterpumpen für den medizinischen Anwendungsbereich zu, die oft radial komprimierbar und expandierbar sind, um sie durch einen Katheter bzw. durch Hohlräume im Körper eines Patienten an einen Anwendungsort befördern und dort expandieren zu können, bevor die Inbetriebnahme erfolgt. Solche Pumpen werden beispielsweise zur Unterstützung eines Herzens eines Patienten bei der Blutförderung eingesetzt und zu diesem Zweck durch ein Blutgefäß bis an oder in eine Herzkammer vorgeschoben.

Dabei ergeben sich besondere Herausforderungen durch die geringe Größe des Rotors und zusätzlich seine Komprimierbarkeit. Im expandierten Zustand muss der Rotor trotz seiner Komprimierbarkeit reproduzierbar eine Betriebsform einnehmen, die sich auch im Betrieb bei möglichst hohen Förderdrehzahlen so wenig wie möglich ändert, um eine Verringerung der Effizienz sowie eine Schädigung der zu fördernden Blutbestandteile zu verhindern.

Aus diesem Grund ist bereits der Einsatz verschiedenster Materialien und Materialkombinationen zu dem genannten Zweck erwogen und untersucht worden. Beispielsweise ist aus der WO 2010/063494 A1 der Einsatz verschiedenster Elastomere, auch im Zusammenhang mit einer Faserverstärkung, bereits bekannt.

Aus der WO 2012/007141 A1 ist eine Verstärkung eines Pumpenrotors durch Fasern bekannt, die in orientierter Form, beispielsweise in Radialrichtung, im Rotor angeordnet sein können.

Schließlich ist aus der WO 2012/007140 A1 ein Pumpenrotor mit Verstärkungselementen bekannt, die im Wesentlichen außerhalb der Förderelemente, beispielsweise auf deren Oberflächen, vorgesehen sein können.

Ein weiterer relevanter Stand der Technik ist aus US 2013/0177409 A1 bekannt.

Vor dem Hintergrund des Standes der Technik liegt der vorliegenden Erfindung somit die Aufgabe zugrunde, einen Kunststoffrotor der oben genannten Art zu schaffen, der eine minimale Relaxation nach Verformungen zwischen dem komprimierten und dem expandierten Zustand sowie eine möglichst genaue Reproduzierbarkeit seiner Geometrie zumindest im Betriebszustand aufweist.

Die Aufgabe wird durch einen Rotor, ein Verfahren zur Herstellung eines Rotors sowie eine entsprechende Gießform für einen Rotor gelöst.

Die Erfindung ist durch den unabhängigen Anspruch 1 definiert. Die abhängigen Ansprüche 2 bis 15 betreffen besondere Ausführungsformen.

Derartige Fluidpumpen werden üblicherweise im komprimierten Zustand durch eine Schleuse in ein Blutgefäß, beispielsweise eine Arterie, eingeführt und bis in die Nähe des Herzens oder teilweise bis in ein Patientenherz hinein eingeschoben, um dort expandiert zu werden. Die Erfindung beschränkt sich jedoch nicht auf derartige Blutpumpen, sondern umfasst auch andere Arten von Blutpumpen mit komprimierbaren und expandierbaren Rotoren oder andere Fluidpumpen, die für medizinische oder nichtmedizinische Zwecke in Systeme von Hohlräumen einführbar sind. Im komprimierten Zustand steht der Rotor einer solchen Pumpe üblicherweise unter einer radialen Kompressionsspannung, indem er beispielsweise durch eine äußere Hülle zusammengehalten wird. Die äußere Hülle kann beispielsweise ein Katheter sein, aus dem die Pumpe zur Expansion hinausgeschoben wird. Der Rotor der Pumpe kann jedoch auch durch jede andere Art äußerer Hülle im komprimierten Zustand zusammengehalten werden.

Wird die komprimierende Kraft eliminiert, so entspannt sich die Pumpe, oder zumindest der Rotor der Pumpe, und geht in einen zweiten, expandierten entspannten Zustand über, in dem die radiale Ausdehnung des Rotors sich gegenüber dem ersten, komprimierten Zustand erheblich erweitert.

Wird dann die Pumpe in Betrieb genommen, indem der Rotor rotatorisch angetrieben und ein Fluid gefördert wird, so geht der Rotor in einen Betriebszustand über, der einem Lastzustand unter der Last der geförderten Flüssigkeit entspricht. Besonders dieser Betriebszustand soll bei einer definierten geometrischen Gestalt des Rotors stattfinden, die von der Höhe der Last möglichst unabhängig sein soll. Der Rotor soll zudem beim Übergang zwischen dem zweiten und dem dritten Zustand eine möglichst geringe Formänderung durchlaufen. Jedoch kann der Rotor im dritten Zustand unter der Last der geförderten Flüssigkeit derart verformt werden, dass der Durchmesser des Rotors sich gegenüber dem zweiten, expandierten Zustand vergrößert (nicht beansprucht).

Die strangförmigen Verstärkungselemente/Fasern, die das Kunststoffmaterial des Rotors verstärken, sollen derart in der Matrix des Kunststoffmaterials angeordnet sein, dass sie im dritten Zustand wenigstens abschnittsweise gestreckt verlaufen und dadurch den Rotor in seiner Form gegenüber der Last stabilisieren. Die Fasern sollen dabei in Längsrichtung zugbeansprucht werden. Da die Fasern in Längsrichtung praktisch nicht dehnbar sind, führen entsprechende Belastungen in Längsrichtung der Fasern kaum zu einer merklichen Formveränderung der Matrix. Der Rotor oder einzelne Elemente des Rotors, beispielsweise die Förderelemente, kann/können sich somit in der Form vom komprimierten Zustand bis zum Betriebszustand problemlos so lange verändern, bis die Fasern wenigstens abschnittsweise gestreckt sind. An diesem Punkt wird durch die Verankerung der Fasern im Kunststoffmaterial des Rotors zumindest in dem Bereich, in dem die Fasern gestreckt sind, eine Bewegung in Längsrichtung der Fasern auch unter Last praktisch unmöglich gemacht.

Eine besondere Ausgestaltung der Lösung sieht beispielsweise dabei vor, dass die Verstärkungselemente/Fasern im dritten Zustand (Betriebszustand) des Rotors wenigstens abschnittsweise in Bereichen des Rotors, in denen dieser eine Dehnspannung aufweist, gestreckt und im Wesentlichen in Richtung der Dehnspannung verlaufen.

Die Verstärkungselemente/Fasern sollten wenigstens abschnittsweise in der Verlaufsrichtung angeordnet werden, in der Dehnspannungen im Rotor auftreten, so dass diese Dehnspannungen durch Aufnahme der Längskräfte in den Fasern abgefangen werden und der Rotor sich in seiner Form praktisch nicht bzw. nicht weiter verändert.

Dies erfordert beispielsweise, dass wenigstens ein Teil der Verstärkungselemente/Fasern außerhalb der sogenannten neutralen Faser in den Förderelementen des Rotors verläuft. Im Bereich der sogenannten neutralen Faser und/oder neutralen Fläche in der Nomenklatur der Biegemechanik würde eine zugfeste Faser keine zusätzliche Stabilisierung des Rotors erbringen. Die biegetechnisch neutrale Faser ist bei den hier beschriebenen Rotoren in der Regel eine Fläche, weshalb im Folgenden zur besseren Klarheit der Begriff biegeneutrale Fläche bzw. neutrale Fläche verwendet wird.

Es kann deshalb vorgesehen sein, dass mehr als die Hälfte der Verstärkungselemente/Fasern in Bereichen des Rotors und/oder der Förderelemente angeordnet sind, in denen dieser/diese Im Betriebszustand Dehnspannungen aufweist/aufweisen. Besonders vorteilhaft ist es dabei, wenn viele Fasern in dem Bereich des Rotors oder eines Förderelements angeordnet sind, der unter Last Dehnspannungen aufweist.

Zudem ist vorgesehen, dass bei dem Rotor zwischen dem zweiten, expandierten Zustand in dem dritten Betriebszustand bezüglich der äußeren Form im Wesentlichen keine Unterschiede bestehen.

Zu einer besonders guten Stabilisierung des Rotors insgesamt kann beispielsweise auch vorgesehen sein, dass Verstärkungselemente/Fasern vorgesehen sind, die sich in radialer Richtung über die Rotationsachse hinaus erstrecken. Damit werden einzelne Förderelemente nicht nur gegen eine Biegung in sich stabilisiert, sondern auch gegenüber einer Schwenkbewegung um einen rotationsachsennahen Teil des Rotors, beispielsweise eine Nabe.

Zudem kann vorteilhaft vorgesehen sein, dass im Betriebszustand wenigstens ein Teil der Fasern, insbesondere die Mehrheit der Verstärkungselemente/Fasern, abschnittsweise gerade verlaufen. Zwischen den Abschnitten, in denen die Verstärkungselemente/Fasern gerade verlaufen, können andere gerade Abschnitte oder auch gekrümmte Abschnitte der Fasern vorgesehen sein.

Es kann auch vorgesehen sein, dass im Betriebszustand wenigstens ein Teil der Fasern, insbesondere die Mehrheit der Verstärkungselemente/Fasern, entlang der Längsrichtung des jeweiligen Förderelementes mit einer geringeren Krümmung verlaufen als die neutrale Fläche des jeweiligen Förderelementes im Sinne der Festigkeitslehre. Durch diese Anordnung der Verstärkungselemente/Fasern wird sichergestellt, dass die Verstärkungsfasern einerseits im Betriebszustand bereits stark gestreckt sind und dass sie andererseits außerhalb der neutralen Fläche der Förderelemente verlaufen und somit in solchen Bereichen, die unter Belastungen Dehnspannungen aufweisen können.

Es kann zudem auch vorgesehen sein, dass der Kunststoff des Rotors eine Shore-Härte <100 D, insbesondere < 80 D, aufweist. Durch die geringe Shore-Härte, die beispielsweise auch kleiner als 80 D gewählt werden kann, ergibt sich bei starker Biegung oder Knickung des Materials für die eingebetteten Verstärkungselemente/Fasern die Möglichkeit, in das nachgiebige Material der Basis auszuweichen und dadurch den Krümmungsradius nach unten zu begrenzen. Damit ergibt sich eine Verringerung der Bruchgefahr für die Elemente/Fasern.

Zudem kann bei einem solchen Rotor auch vorgesehen sein, dass ein erster Anteil von mehr als 30%, insbesondere mehr als 50%, der Verstärkungselemente/Fasern im expandierten Zustand des Rotors im Wesentlichen gestreckt von einem der Rotorachse am nächsten liegenden Abschnitt zu einem der Rotationsachse ferner liegenden zweiten Abschnitt verläuft. Durch diese Positionierung, Ausrichtung und Formung der Verstärkungselemente/Fasern wird eine Überstreckung des Rotors über den expandierten Zustand hinaus wirkungsvoll vermieden.

Eine Rotorkonstruktion kann auch dadurch ausgestaltet werden, dass ein erster Anteil der Verstärkungselemente/Fasern, der mehr als 30% der Verstärkungselemente/Fasern, insbesondere mehr als 50%, der Verstärkungselemente/Fasern beträgt, eine Länge aufweist, die mindestens 30%, insbesondere mindestens 50%, der Maximalhöhe der Förderelemente, gemessen in Radialrichtung des Rotors im expandierten Zustand, entspricht. Hierdurch ergeben sich Stabilisierungskräfte, die größere Teile des Rotors umfassen und diesen insgesamt stabilisieren.

Dazu kann außerdem vorgesehen sein, dass der erste Anteil der Verstärkungselemente/Fasern im Betriebszustand im Wesentlichen senkrecht zur Rotationsachse verläuft. Durch diese Ausrichtung der Verstärkungselemente/Fasern können Dehnspannungen im Rotor, die durch die Last des zu fördernden Fluids erzeugt werden, optimal aufgenommen werden.

Weiter kann die Bauart des Rotors dadurch ausgestaltet werden, dass der Durchmesser der Verstärkungselemente/Fasern, insbesondere des ersten Anteils der Verstärkungselemente/Fasern, kleiner als 40 Mikrometer ist. Durch einen derart klein gestalteten Durchmesser der Verstärkungselemente/Fasern können geringe Biegeradien erreicht werden, so dass die Bruchgefahr für die Verstärkungselemente/Fasern weiter vermindert ist.

Zudem kann vorgesehen sein, dass die Oberfläche der Verstärkungselemente/Fasern mit einem Haftvermittler versehen ist. Dadurch wird eine gute Verankerung der Verstärkungselemente/Fasern im Kunststoffmaterial der Matrix erreicht, was zu einer weiteren Verbesserung bei der Aufnahme von Dehnspannungen durch die Verstärkungselemente/Fasern führt.

Die Offenbarung bezieht sich zudem auf ein Verfahren zur Herstellung eines Rotors mittels eines Gießverfahrens, insbesondere eines Spritzgießverfahrens, bei dem das Material der Förderelemente in Radialrichtung in Bezug auf die Rotorachse in die Volumina der Förderelemente derart eingebracht wird, dass der Gießwerkstoff in das Volumen jedes einzelnen Förderelementes jeweils in Radialrichtung einströmt.

Bei einem solchen Verfahren kann das Gussmaterial beispielsweise in Axialrichtung im zentralen Bereich des Rotors, beispielsweise im Volumen einer Rotornabe, eingespritzt und von dort radial in die einzelnen Förderelemente hinein verteilt werden. Jedes einzelne Förderelement wird dann in einer radial gerichteten Flussrichtung des Materials von innen nach außen gefüllt. Es ist jedoch auch eine Füllung von den radial außen liegenden Enden der Förderelemente radial nach innen, auf die Rotorachse hin gerichtet, möglich.

Die in dem Gussmaterial vorhandenen Verstärkungselemente/Fasern oder auch Folienabschnitte, die beispielsweise aus Glas, jedoch auch aus Kohlenstoff bzw. Polycarbonat oder Metall bestehen können, werden durch den Strom des Matrixmaterials mitgenommen und vorwiegend in Flussrichtung des Materials ausgerichtet.

Eine für die Herstellung des beschriebenen Rotors und für das beschriebene Verfahren optimierte Gießform soll ebenfalls vorgestellt werden. Diese zeichnet sich dadurch aus, dass bei den Volumina der Förderelemente an deren radial verlaufenden Kanten Überströmkanäle vorgesehen sind, um einen störungsfreien Fluss des Gießwerkstoffs in Radialrichtung zu ermöglichen.

Durch die an den Kanten vorgesehenen Überströmkanäle wird die Ausbildung von Verwirbelungen mit lokalen Rückströmungen im Bereich der Kanten der Förderelemente verhindert, so dass eine weitgehend laminare Strömung ausgebildet werden kann. Hierdurch werden die Verstärkungselemente/Fasern in überwiegend gestreckter Lage in die Matrix eingebettet. Dies ist insbesondere deshalb sinnvoll, da die Gießform den Guss eines Rotors im zweiten, expandierten Zustand, d. h. frei von äußeren Spannungen, ermöglicht.

Zusätzlich zu den an den seitlichen Kanten der Förderelemente vorgesehenen Überströmkanälen können auch an den radial außen gelegenen Enden der Volumina in der Gießform Überströmkanäle ausgebildet sein, die das Abströmen an den radial außen gelegenen Enden der Förderelemente erlauben. Das überschüssige Gussmaterial kann nach wenigstens teilweisem Erstarren des Rotors in der Gießform entfernt werden.

Es kann bei der Anordnung der Verstärkungselemente/Fasern im Volumen der Förderelemente gezielt eine Beabstandung von der biegeneutralen Fläche angestrebt werden, um bei der Belastung durch den Fluidgegendruck im Betriebszustand eine besondere Stabilisierung bei Ausbiegung der Förderelemente in eine Richtung durch Streckung der Verstärkungselemente/Fasern zu erreichen. Jedenfalls sollten die Verstärkungselemente/Fasern vorteilhaft im Inneren der Förderelemente verlaufen und einen bestimmten Mindestabstand zu den äußeren Begrenzungsflächen der Förderelemente einhalten.

Es können in dem Rotor auch mehrere Gruppen von Verstärkungselementen/Fasern unterschiedlicher Länge vorgesehen sein, wobei wenigstens eine Gruppe eine bestimmte Mindestlänge aufweist, während die Verstärkungselemente/Fasern von einer oder mehreren Gruppen kürzer sind oder eine Längenverteilung aufweisen, die nur vernachlässigbar wenige Verstärkungselemente/Fasern oberhalb einer Faserlänge aufweist, die unterhalb einer typischen Länge des ersten Anteils der Verstärkungselemente/Fasern liegt. Typisch liegt die Durchschnittslänge der kürzeren Verstärkungselemente/Fasern bei weniger als einem Drittel der Länge des ersten Anteils der Verstärkungselemente/Fasern.

Der Rotor kann vorteilhaft dadurch ausgestaltet werden, dass jedes Verstärkungselement / jede Faser des ersten Anteils der Verstärkungselemente/Fasern in ihrem Verlauf höchstens 45° in Axialrichtung und/oder Azimutalrichtung von einer radial auf die Rotorachse (14) ausgerichteten Lage abweicht. Die Verstärkungselemente/Fasern verlaufen dann in einer Fläche, in der auch die gesamte Rotationsachse des Rotors verläuft, so dass sie sich beispielsweise direkt von der Rotationsachse ausgehend zunächst senkrecht radial nach außen erstrecken können. Jedoch ist auch eine Ausrichtung der Verstärkungselemente/Fasern von der Rotationsachse radial in einem Winkel zwischen 45° und 90° zur Rotationsachse möglich. In einer Ausgestaltung weist die Erstreckung der Verstärkungselemente/Fasern jedenfalls keine oder nur geringe azimutale (in Umfangsrichtung verlaufende) Ausrichtung auf.

Der Gegenstand kann weiterhin dadurch ausgestaltet sein, dass die Förderelemente aus einem Schaumstoff bestehen. Dabei ist insbesondere an einen geschlossenporigen Schaumstoff gedacht, der durch die Verstärkungsfasern effektiv stabilisierbar ist und der dennoch einfach und in ausreichendem Maß komprimierbar ist. Bei der Kompressionsbewegung ist gerade bei einem Schaumstoff ein Ausweichen der Verstärkungselemente/Fasern zur Verhinderung der Unterschreitung eines kritischen Biegeradius besonders einfach möglich. Üblicherweise weisen derartige Schaumstoffe im Inneren des Volumens der Förderelemente entsprechende Poren auf, sind jedoch an den äußeren Begrenzungsflächen praktisch vollständig geschlossen.

Der Rotor kann auch derart gestaltet sein, dass ein Anteil von Verstärkungselementen/Fasern im expandierten Zustand des Rotors zu den Verstärkungselementen/Fasern des ersten Teils quer verläuft und insbesondere mit diesen im Mittel Winkel von mindestens 30° einschließt. Dadurch kann die jeweilige Gruppe von Fasern ein Biegen des Rotors oder eines Förderelements des Rotors jeweils in Längsrichtung der Fasern fast vollständig verhindern, sofern die Biegung in einer Richtung erfolgt, in der die Fasern auf Zug beansprucht werden. Sind in der beschriebenen Art zwei Richtungen durch die verschiedene Anordnung von zwei Fasergruppen ausgezeichnet, so lässt sich eine dreidimensionale Form des Rotors oder eines Teils des Rotors in sehr effizienter Weise stabilisieren und gegen Biegung in verschiedenen Richtungen verfestigen.

Es kann zudem vorgesehen sein, dass die Verstärkungselemente/Fasern wenigstens teilweise in Form von Gewebeabschnitten mit längs und quer verlaufenden Fasern vorliegen. Die Gewebeabschnitte können sich beispielsweise in ihrer Längsrichtung wenigstens zwei-, drei-, fünf- oder zehnmal so weit erstrecken wie in der senkrecht dazu liegenden Querrichtung, so dass sie jeweils einen länglichen Streifen bilden. Innerhalb dieser Gewebe sind dann ohne weiteres erste Fasern in einer Längsrichtung und dazu quer verlaufende zweite Fasern senkrecht oder in einem stumpfen Winkel dazu vorgesehen.

Es kann beispielsweise auch vorgesehen sein, dass die Verstärkungselemente in Form von Folienstreifen vorliegen, deren Länge wenigstens dreimal, insbesondere wenigstens fünfmal, weiter insbesondere wenigstens zehnmal so groß ist wie ihre Breite. Diese Folienstreifen können aus einem anisotropen Polymer bestehen, das beispielsweise in Längsrichtung wesentlich zugfester ist als in Querrichtung. Sie können jedoch auch aus einer isotropen Folie bestehen, beispielsweise aus zugfestem Kunststoffmaterial oder auch aus einem Metall, wie Aluminium, Silber, Titan, Nitinol oder Gold,

Erfindungsgemäß ist vorgesehen, dass die Verstärkungselemente von dem Kunststoff, aus dem der Rotor überwiegend besteht, wenigstens auf einem Anteil von 90%, insbesondere 99 %, ihrer Oberfläche, weiter insbesondere vollständig, umschlossen sind. Es kann im Einzelfall geschehen, dass Verstärkungselemente in der Spritzgießform an deren Wand stoßen, so dass sie im fertigen Produkt an die äußere Oberfläche des Rotors treten. Dabei sind dann jedoch im Normalfall nur die Enden der Verstärkungselemente an der Außenseite des Rotors freiliegend angeordnet, wobei auch dies durch das Einbringen von Verstärkungselementen und eine geeignete Strömungsführung beim Spritzgießen während des Spritzgießvorgangs relativ unwahrscheinlich wird.

Es kann bei dem Rotor außerdem auch vorgesehen sein, dass das Kunststoffmaterial, das die Verstärkungselemente einbettet, auf der im Betrieb von dem Fluidgegendruck nicht belasteten Seite der Förderelemente wenigstens bereichsweise andere Eigenschaften aufweist als auf der mit dem Fluidgegendruck belasteten Seite der Förderelemente, insbesondere auf der von dem Fluidgegendruck nicht belasteten Seite stärker vernetzt oder geschrumpft ist oder dort auf der Oberfläche eine auf dem Förderelement geschrumpfte Auflage in Form von einer oder mehreren Folien, Beschichtungen oder Fasern trägt. Mit den beiden Seiten der Förderelemente sind in diesem Zusammenhang die Volumenbereiche auf den beiden Seiten der im Pumpbetrieb bei der Biegebelastung biegeneutralen Ebene oder Fläche im Volumen des jeweiligen Förderelementes gemeint.

Eines der Ziele, die mit der Erfindung angestrebt werden können, ist, dass der zweite Zustand des Rotors, den dieser im von außen kraftfreien Zustand einnimmt, sich möglichst wenig von einem dritten Zustand unterscheidet, den der Rotor im Betrieb während seiner Rotation in einem Fluid unter der Wirkung des Fluidgegendrucks einnimmt. Deshalb ist erfindungsgemäß vorgesehen, dass die Verstärkungsfasern bereits im zweiten Zustand des Rotors möglichst weitgehend gestreckt sind und in einer Richtung verlaufen, in der sie zumindest Biegungen des Rotors begrenzen.

Dies kann dadurch unterstützt werden, dass die Form des Rotors in dem zweiten Zustand, in dem er sich kraftfrei befindet, sich von der Form, die der Rotor in der Spritzgießform einnimmt, unterscheidet. Es kann durch geeignete Gestaltung der Kunststoffmatrix, die durch den Spritzgießwerkstoff gebildet wird, bewirkt werden, dass bereits im von außen kräftefreien Zustand durch elastische Kräfte des Spritzgießwerkstoffs eine Verformung in Richtung auf den dritten Zustand hin stattfindet, die die Fasern vorspannt. Dieser Effekt kann beispielsweise dadurch erreicht werden, dass ein von den Verstärkungselementen unterschiedliches Material im oder am Rotor vorgesehen ist, dass den Rotor, insbesondere die Förderelemente, in eine Form bringt, in der die Verstärkungselemente vorgespannt sind. Da beim eigentlichen Vergießen der Verstärkungselemente in der Matrix diese mehr oder weniger kraftfrei vorliegen, gelingt dies durch eine Veränderung der Kunststoffmatrix oder des Körpers des Rotors insgesamt nach dem Abschluss des Spritzgießvorgangs. Beispielsweise kann die Kunststoffmatrix nach der Entnahme aus der Spritzgussform besonders behandelt werden, derart, dass das Material der Förderelemente auf der Seite, die dem Fluidgegendruck während des Betriebs ausgesetzt ist, gelängt oder auf der gegenüberliegenden Seite geschrumpft bzw. anisotrop verkürzt wird. Dies kann beispielsweise durch eine ungleichmäßige Vernetzung der Kunststoffmatrix geschehen, die auf den beiden Seiten der neutralen Faser der Förderelemente unterschiedlich ist. Es kann jedoch auch dadurch gelingen, dass die Förderelemente auf der Seite, die der im Betrieb dem Fluidgegendruck ausgesetzten Seite der Förderelemente gegenüberliegt, mit einer Folie beschichtet werden, die nach der Beschichtung schrumpft oder geschrumpft werden kann, beispielsweise durch Elektronenstrahlvernetzung oder UV-Vernetzung oder durch thermische Behandlung.

Es kann auch ein Rotor für eine komprimierbare Fluidpumpe vorliegen, der radial zwischen einem ersten, komprimierten und einem zweiten, expandierten Zustand expandierbar und komprimierbar ist, wobei zudem vorgesehen sein kann, dass ein oder mehrere Förderelemente des Rotors durch Spritzgießen mit gleichzeitigem Hinzufügen von Verstärkungselementen im expandierten Zustand hergestellt ist, wobei die Verstärkungselemente durch einen Spritzgießwerkstoff allseitig umschlossen sind und wenigstens abschnittsweise im expandierten Zustand gestreckt, insbesondere zu wenigstens 90 %, weiter insbesondere zu 95 %, weiter insbesondere zu 99 % gestreckt bzw. bei Verwendung eines Gewebes soweit wie möglich gestreckt vorliegen.

Der Rotor kann beispielsweise auch derart gestaltet sein, dass die Verstärkungselemente im zweiten, expandierten Zustand des Rotors ohne einen Fluidgegendruck in einem solchen Maß gestreckt vorliegen, dass sie sich beim Übergang zu einem dritten Zustand, der den Betriebszustand mit einem Fluidgegendruck darstellt, um weniger als 5 %, insbesondere weniger als 1 %, verlängern, wobei die Verlängerung insbesondere am Abstand der beiden Enden eines Verstärkungselementes voneinander vor und während der Zugbelastung bemessen ist.

Es kann beispielsweise bei dem Rotor weiter vorgesehen sein, dass in einem zweiten, expandierten Zustand des Rotors und/oder einem dritten Betriebszustand mit Fluidgegendruck wenigstens ein Anteil der Verstärkungselemente, insbesondere mindestens 10 %, weiter insbesondere mindestens 30 %, in wenigstens einem Bereich eines Förderelementes, in dem dieses gekrümmt ist, gestreckt und gerade verlaufen, soweit von den Ansprüchen umfasst.

Es kann außerdem vorgesehen sein, dass in einem Bereich eines Förderelementes, in dem dieses gekrümmt ist, wenigstens zwei Anteile von Verstärkungselementen gerade und gestreckt verlaufen, wobei die Richtungen, in denen die Verstärkungselemente verlaufen, bei dem jeweiligen Anteil parallel, jedoch bei den verschiedenen Anteilen verschieden sind. Die Fasern der verschiedenen Gruppen können dabei bereits miteinander verbunden als Gewebe oder getrennt voneinander, insbesondere nacheinander, in die Spritzgussform eingebracht werden.

Eine weitere Ausgestaltung des Rotors kann vorsehen, dass die Länge der Verstärkungselemente bei wenigstens 30 %, insbesondere wenigstens 50 %, der Elemente größer ist als die durchschnittliche Dicke der Förderelemente, insbesondere wenigstens zweimal so lang, weiter insbesondere wenigstens 5-mal oder 10-mal so lang. Die so gekennzeichneten langen Verstärkungselemente können beim Füllen des Gießwerkstoffs durch andere Füllelemente, wie beispielsweise sehr kurze Fasern und/oder Partikel, ergänzt werden, wobei die kurzen Fasern dabei auch jeweils in gestreckter Form vorliegen können. Dies bewirkt jedoch wenig in Bezug auf eine Begrenzung der Krümmung des Rotors, da diese Fasern üblicherweise sehr kurz sind. Mit der Dicke der Förderelemente ist in diesem Zusammenhang an jedem Punkt der Förderelemente die Ausdehnung in derjenigen Richtung gemeint, in der die Ausdehnung des jeweiligen Förderelements am geringsten ist.

Eine weitere Ausgestaltung des Rotors kann vorsehen, dass Verstärkungselemente, insbesondere Fasern, in den sie einbettenden Kunststoff in einem Spritzgussverfahren eingebracht sind und während der Zeit, in der sich der Rotor in der Spritzgussform befindet, einen abschnittsweise gekrümmten Verlauf entlang der Strömung des Kunststoffs in die Spritzgussform aufweisen.

Die Offenbarung bezieht sich zudem auch auf eine Gießform für einen Rotor der oben beschriebenen Art, bei der vorgesehen ist, dass bei den Volumina der Förderelemente an deren radial verlaufenden Kanten Überströmkanäle vorgesehen sind, um einen störungsfreien Fluss des Gießwerkstoffs in Radialrichtung zu ermöglichen.

Ein Verfahren zur Herstellung des oben beschriebenen Rotors kann vorsehen, dass das Material der Förderelemente in Radialrichtung in Bezug auf die Rotorachse in die Volumina der Förderelemente derart eingebracht wird, dass der Gießwerkstoff in das Volumen jedes einzelnen Förderelementes jeweils in Radialrichtung einströmt.

Durch Anordnung und Größe der Überströmkanäle der Gießform kann darüber hinaus die Strömungsrichtung des einfließenden Gießwerkstoffes und damit die Ausrichtung der Fasern entlang der Strömung gesteuert werden.

Ein weiteres Verfahren zur Herstellung eines Rotors kann vorsehen, dass der Rotor durch Gießen, insbesondere Spritzgießen, hergestellt wird, wobei das Spritzgießverfahren in zwei aufeinanderfolgenden Phasen mit unterschiedlichen Einspritzrichtungen und/oder von zwei verschiedenen Einspritzstellen aus durchgeführt wird. Hierdurch können gezielt Verstärkungselemente/Fasern in den Spritzgießwerkstoff eingebracht werden, die gruppenweise in unterschiedlichen Richtungen verlaufen.

Es kann auch bei einem Verfahren zur Herstellung eines Rotors vorgesehen sein, dass der Rotor nach dem Spritzgießen einer Behandlung unterworfen wird, die eine unterschiedliche Schrumpfung und/oder Vernetzung des Gussmaterials auf der im Betrieb durch den Fluidgegendruck belasteten Seite der Förderelemente als auf der dieser gegenüberliegenden Seite bewirkt. Hierdurch kann der Rotor durch Erzeugung innerer Spannungen derart vorgespannt werden, dass er ohne Einwirkung äußerer Kräfte bereits den dritten Zustand, d. h. einen Betriebszustand, einnimmt, der stabil ist, wenn der Rotor einem Fluidgegendruck ausgesetzt ist. Dies geschieht dadurch, dass die inneren Spannungen derart gerichtet und bemessen sind, dass hierdurch die Verstärkungselemente bereits mit einer Zugkraft beaufschlagt werden, die in der Größenordnung der Kräfte liegt, denen die Verstärkungsfasern im Betrieb des Rotors ausgesetzt sind.

Hierzu kann beispielsweise auch vorgesehen sein, dass auf der der im Betrieb einem Fluidgegendruck ausgesetzten Seite der Förderelemente gegenüberliegenden Seite auf wenigstens eines der Förderelemente eine schrumpfbare oder schrumpfende Schicht aufgebracht wird.

Alternativ oder zusätzlich kann beispielsweise vorgesehen sein, dass bei der Gießform wenigstens zwei verschiedene Einspritzöffnungen für den Spritzgießwerkstoff vorgesehen sind. Dadurch kann während des Gießvorgangs die Strömung des Spritzgießwerkstoffs in der Form gezielt verändert werden, so dass bei Zusatz von Verstärkungselementen in verschiedenen Phasen die Verstärkungselemente jeweils in der Hauptströmungsrichtung des Spritzgießwerkstoffs und damit in unterschiedlichen Richtungen entsprechend den verschiedenen Phasen des Spritzgießvorgangs angeordnet werden können. Hierzu kann beispielsweise eine erste Teilmenge des Spritzgießwerkstoffs durch eine erste Einspritzöffnung und eine zweite Teilmenge durch eine zweite Einspritzöffnung nacheinander oder gleichzeitig oder in einem veränderlichen Mengenverhältnis zueinander eingespritzt werden.

Im Folgenden wird die Neuerung anhand von Ausführungsbeispielen in Figuren einer Zeichnung gezeigt und nachfolgend erläutert. Dabei zeigt
- Fig. 1: schematisch in einem Schnitt eine Katheterpumpe, die in eine Herzkammer eines Patienten eingeführt ist,
- Fig. 2a: einen Ausschnitt aus einem Rotor einer Katheterpumpe zur Förderung von Blut,
- Fig. 2b: eine Querschnittsdarstellung eines entspannten Rotors mit einer beispielhaft dargestellten Faser,
- Fig. 2c: die Querschnittsdarstellung eines Rotors im dritten Zustand, dem Betriebszustand,
- Fig. 2d: die Querschnittsdarstellung eines Rotors im komprimierten Zustand,
- Fig. 2e: eine Querschnittsdarstellung eines Förderelementes mit Markierung der biegetechnisch neutralen Fläche sowie mit beispielhaft dargestellten Verstärkungsfasern,
- Fig. 3: eine schematische Darstellung einer Ebene, die die Rotationsachse des Rotors enthält,
- Fig. 4: einen Ausschnitt aus einem planen Förderelement mit einer Faser zur Verstärkung,
- Fig. 5a: die Ausrichtung einer Faser beim Gießvorgang,
- Fig. 5b: eine Seitenansicht des Abschnitts aus Figur 4,
- Fig. 6: den Abschnitt eines Förderelements wie in Figur 4 und 5 gezeigt nach einem Knickvorgang,
- Fig. 7: eine schematische Ansicht eines Gießwerkzeugs für einen Rotor, wobei der Gießvorgang durch Strömungspfeile angedeutet ist,
- Fig. 8: eine Gießform für einen Rotor mit einer verglichen mit Figur 7 umgekehrten Einspritzrichtung,
- Fig. 9: eine Gießform, bei der Maßnahmen gegen eine Verwirbelung des Spritzgusswerkstoffs im Volumen der Förderelemente getroffen sind,
- Fig. 10: eine weitere Gießform,
- Fig. 11: ein folienartiges, bandförmiges Verstärkungselement,
- Fig. 12: ein gewebeartiges Verstärkungselement sowie
- Fig. 13: einen Querschnitt durch ein Förderelement,
- Fig. 14a: ein Gewebe aus Fasern in einem kraftfreien Zustand,

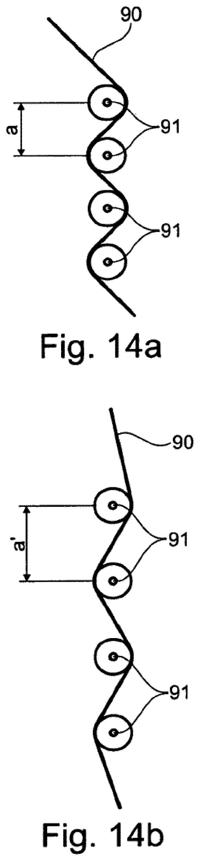

- Fig. 14 b: das Gewebe aus Fig. 14a in einem maximal gestreckten Zustand unter Last,

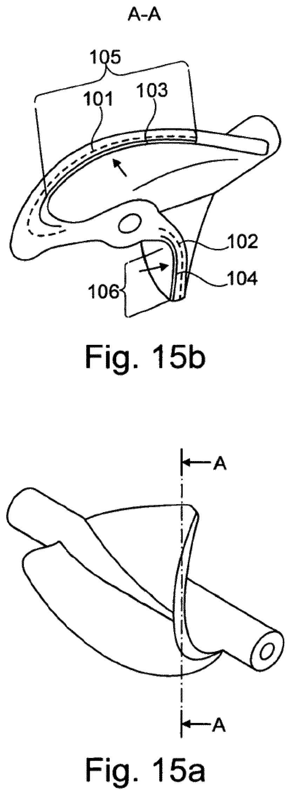

- Fig. 15a: ein Förderelement in einer perspektivischen Ansicht im expandierten Zustand ohne äußere Krafteinwirkung,
- Fig. 15 b: eine Schnittansicht des Förderelements aus Fig. 15a in der dort mit A-A bezeichneten Schnittebene,

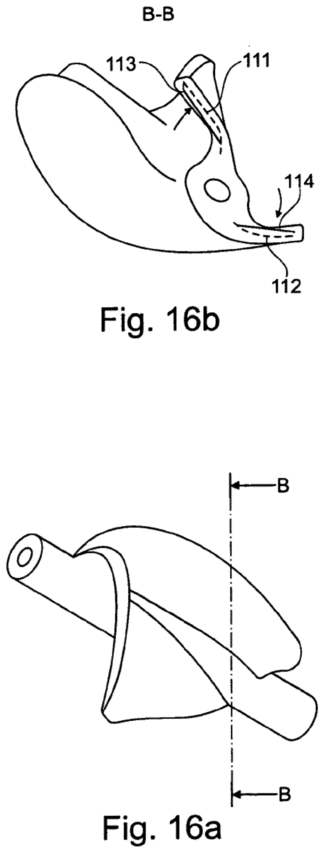

- Fig. 16a: ein Förderelement in einer perspektivischen Ansicht im expandierten Zustand ohne äußere Krafteinwirkung,
- Fig. 16 b: eine Schnittansicht des Förderelements aus Fig. 16a in der dort mit B-B bezeichneten Schnittebene,

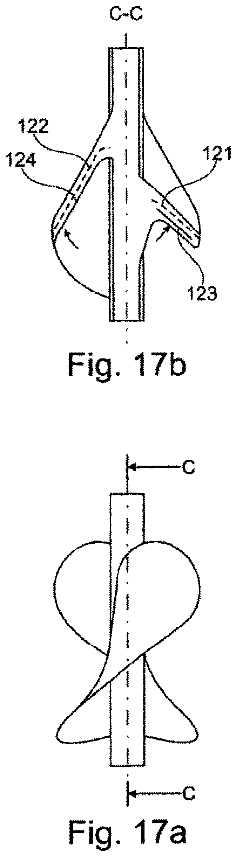

- Fig. 17a: ein Förderelement in einer seitlichen Ansicht im expandierten Zustand ohne äußere Krafteinwirkung und
- Fig. 17b: eine Schnittansicht des Förderelements aus Fig. 17a in der dort mit C-C bezeichneten Schnittebene.

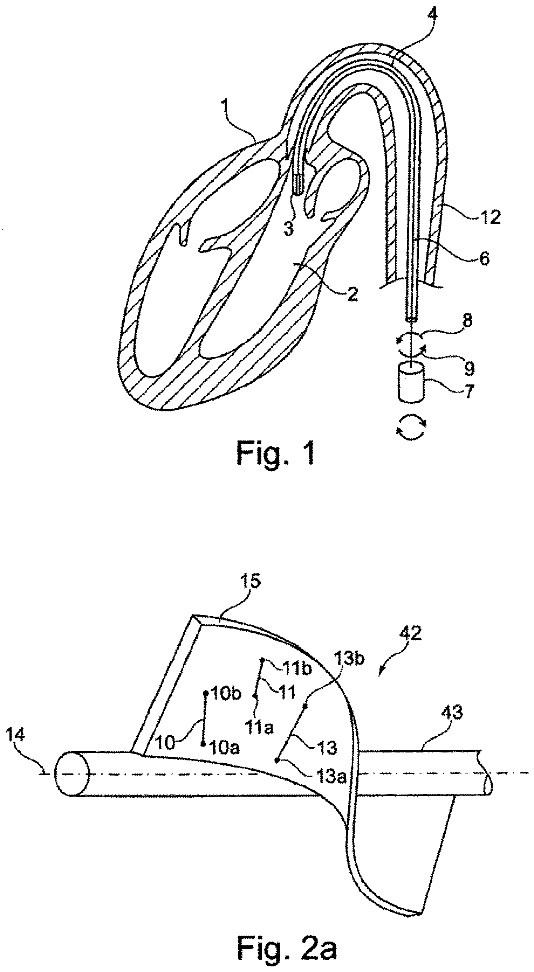

Figur 1 zeigt im Querschnitt das Herz 1 eines Patienten mit mehreren Herzkammern, wobei die Herzkammer 2 mit der Aorta 12 in Verbindung steht. Durch die Aorta 12 ist ein Katheter 4 bis in die Herzkammer 2 vorgeschoben, wobei am Ende des Katheters 4 ein Pumpenkopf 3 mit einer Rotationspumpe angeordnet ist. Die Rotationspumpe ist mittels einer durch den Katheter 4 verlaufenden drehbaren Welle 6 antreibbar, die innerhalb des Pumpenkopfes 3 mit einem Pumpenrotor 42 verbunden ist. Der Pumpenrotor rotiert in einem nicht im Einzelnen dargestellten Gehäuse des Pumpenkopfes 3.

Die biegsame Welle 6 ist mit einem Motor 7 verbunden, der beispielsweise außerhalb des Patientenkörpers angeordnet ist. Das Drehmoment kann beispielsweise über eine Magnetkopplung von dem Motor 7 in beiden Drehrichtungen 8, 9 auf die Welle 6 übertragen werden.

Der Katheter 4 wird üblicherweise über eine Schleuse vom Körperäußeren durch die Haut und Gewebe sowie die Gefäßwand in die Aorta 12 hinein und in dieser vorgeschoben.

Die Pumpe saugt in der Herzkammer 2 Blut an und fördert dies in die Aorta 12. Hierdurch kann die Herzpumpe die Funktion des Herzens 1 entweder unterstützen oder zumindest zeitweise ersetzen.

Neben der in der Figur dargestellten Katheterpumpe mit mechanischem Antrieb sind auch andere Pumpen, insbesondere zur intrakorporalen Anwendung, Gegenstand dieses Schutzrechts, beispielsweise Pumpen mit hydraulischem oder elektrischem Antrieb, und dort auch Pumpen, bei denen der Antrieb innerhalb des menschlichen Körpers ist.

Die Pumpe wird mitsamt dem Pumpenkopf, dem Pumpengehäuse und dem Rotor zur Verschiebung in der Aorta radial komprimiert und beispielsweise innerhalb des Katheters 4 verschoben. Die Pumpe kann dann aus dem Katheter 4 axial herausgeschoben werden und sich radial entfalten, d. h. expandiert werden. Dabei werden hohe Anforderungen an die Materialien des Pumpengehäuses und insbesondere des Pumpenrotors gestellt: Die Förderelemente des Pumpenrotors weisen eine sehr geringe Wandstärke auf, müssen aber dennoch auch bei hohen Rotationdrehzahlen formstabil bleiben und in reproduzierbarer Weise Blut fördern.

Zu diesem Zweck sind in die Matrix des Kunststoffs, aus dem der Rotor besteht, Verstärkungsfasern (Fasern) eingebettet, die beispielsweise als Glasfasern oder Polycarbonatfasern ausgestaltet sein können. Solche Verstärkungselemente/Fasern sind in Figur 2a in drei Einzelbeispielen dargestellt. Es sind die drei Verstärkungselemente/Fasern 10, 11, 13 gezeigt, von denen jede ein erstes Ende oder einen ersten Abschnitt 10a, 11a, 13a aufweist, das/der der Rotationsachse 14 näher ist als das jeweilige zweite Ende / der jeweilige zweite Abschnitt 10b, 11b, 13b der Verstärkungselemente/Fasern 10, 11, 13.

Die Verstärkungselemente/Fasern 10, 11, 13 erstrecken sich im Wesentlichen von der Rotationsachse oder einem Punkt in der Nähe der Rotationsachse 14 des Rotors radial nach außen weg. Dabei ist es nicht notwendig, dass der Rotor 42, wie im dargestellten Beispiel, eine Nabe 43 aufweist. Das wendelartige Förderelement 15 kann auch eine derartige Eigenstabilität aufweisen, dass eine Rotornabe nicht notwendig ist.

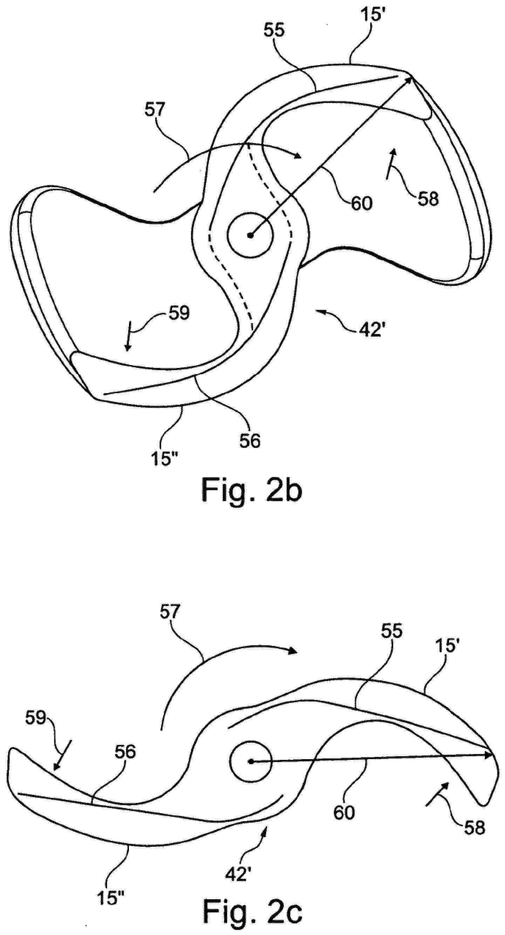

Grundsätzlich kann die Kunststoffmatrix des Förderelements oder der Förderelemente 15 mit Verstärkungselementen/Fasern verstärkt sein, die in Bezug auf Länge und/oder Dicke und/oder Orientierung ungleichmäßig verteilt und angeordnet sind. Ein Aspekt ist, dass ein gewisser Mindestanteil der Verstärkungselemente/Fasern im expandierten Zustand des Rotors, der in Figur 2b dargestellt ist, im Wesentlichen gestreckt von der Rotationsachse weg verläuft. Im Betriebszustand, der dem dritten Zustand entspricht und der in Figur 2c dargestellt ist, sind diese Verstärkungselemente/Fasern gestreckt und stehen zudem unter Zugspannung, die durch die Last der geförderten Flüssigkeit erzeugt wird. Diese Zugspannung führt allerdings wegen der Längsfestigkeit der Verstärkungselemente/Fasern nicht zu einer wahrnehmbaren Längung und somit auch praktisch nicht zu einer Formänderung des Rotors gegenüber dem spannungslosen expandierten Zustand bzw. zu keiner weiteren Verformung oberhalb eines bestimmten Betriebspunktes. Der Anteil der Verstärkungselemente/Fasern, der die genannte Bedingung erfüllt, von der Gesamtmenge der Verstärkungselemente/Fasern, die in den Kunststoff des Rotors eingebettet sind, sollte wenigstens 30% oder vorteilhaft 50% oder noch vorteilhafter beispielsweise 70%, gemessen in Volumen- oder Massenprozent der Verstärkungselemente/Fasern oder auch in der Zahl der Verstärkungselemente/Fasern, darstellen. Dabei ist vorteilhaft eine gewisse Mindestlänge der Verstärkungselemente/Fasern gegeben, beispielsweise etwa wenigstens 20% oder wenigstens 40% oder 50% des Radius des Rotors. Die Verstärkungselemente/Fasern können während des Füllvorgangs der Form leicht aus der axialen Ausrichtung, die der Einfüllrichtung axial in die Nabe 43 entspricht, in die radiale Position der Förderelemente 15 wechseln ohne hierfür gebogen zu werden, da der Winkel zwischen dem Förderelement 15 und der Nabe 43 mit < 30° oder bevorzugt < 20° relativ flach verläuft. Somit werden die Verstärkungselemente/Fasern bei der radialen Befüllung der Förderelemente 15 einfach von der sie umgebenden Matrix mitgenommen und mit dem Materialfluss im Förderelement radial gemäß Figur 2a ausgerichtet.

Weitere vorteilhafte Eigenschaften der Verstärkungselemente/Fasern sind eine gewisse Maximaldicke, wobei ein Durchmesser von höchstens 40 µm vorteilhaft sein kann, um bei einer starken Biegung der Verstärkungselemente/Fasern keinen Bruch der Verstärkungselemente/Fasern selbst zu erzeugen. Mit ca. 40 µm hingegen sind die Verstärkungselemente/Fasern biegesteif genug, um die sie umgebende Matrix nach erfolgter Deformation wieder in den Ausganszustand zurück zu holen und ein nachhaltiges Kriechen der Matrix unter dauerhafter Biegebelastung zu verhindern. Ferner sind Verstärkungselemente/Fasern mit 40 µm Durchmesser druck- und zugsteif, so dass sie bei einer Anordnung außerhalb des biegeneutralen Bereiches ebenfalls ein rückstellendes Moment erzeugen und einer bleibenden Deformation entgegen wirken.

Die Verstärkungselemente/Fasern können für eine Verbesserung der Verbindung mit der Matrix mit einem Haftvermittler beschichtet sein.

Figur 2b zeigt im Querschnitt einen Rotor 42' mit Förderelementen 15', 15" im entspannten, expandierten Zustand. Die Verstärkungselemente/Fasern 55, 56 weisen in diesem Zustand eine möglichst gestreckte Form auf, so dass sie einer weitergehenden Verformung des Rotors durch ihre Längssteifigkeit einen Widerstand entgegensetzen.

Der Pfeil 57 zeigt die Rotationsrichtung des Rotors im Betriebszustand. Die Pfeile 58, 59 zeigen die auf die Förderelemente 15', 15" wirkende Last, d. h. den durch die Förderbewegung wirkenden Fluidgegendruck auf die Förderelemente. In Figur 2b ist die Sehne 60 eingezeichnet, die die Höhe des Förderelements 15' in radialer Richtung von der Rotationsachse aus gemessen bezeichnet.

Die Sehne 60 ist auch in Figur 2c eingezeichnet, welche den Rotor 42' im dritten Zustand, dem Betriebszustand, zeigt. Die Verstärkungsfasern 55, 56 stehen durch die Last 58, 59 unter Zugspannung und nehmen die Dehnungsspannungen in den Förderelementen 15', 15" auf. Die Länge der Sehne 60, d. h. die Höhe der Förderelemente, ist im Betriebszustand gegenüber dem zweiten Zustand gleichbleibend oder um ein geringes Maß erhöht, jedoch nicht verringert.

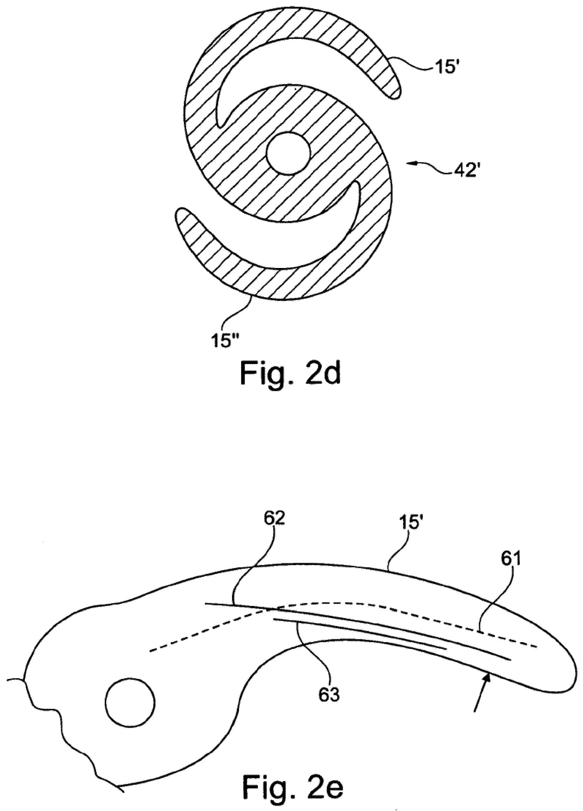

Figur 2d zeigt den Rotor 42' in komprimierter Form, in der die Förderelemente an die Rotornabe angeklappt sind. In dieser Stellung kann die Pumpe durch einen engen Kanal an den Betriebsort gebracht werden.

In Figur 2e ist ein Betriebszustand des Förderelements 15' im Querschnitt detaillierter dargestellt. Es ist die "biegeneutrale Faser" oder "biegeneutrale Fläche" durch die gestrichelte Linie 61 dargestellt, d. h. die Linie oder Fläche, die beim Biegen des Förderelements 15' unter Last weder eine Längung noch eine Stauchung erfährt. Eine Mehrzahl von Verstärkungsfasern, beispielhaft mit 62, 63 bezeichnet, liegt auf der Seite der Linie/Fläche 61, auf der im Betriebszustand eine Dehnspannung entsteht. Die Verstärkungselemente/Fasern sind dort abschnittsweise gestreckt und verlaufen gerade sowie im Wesentlichen parallel zu der Linie/Fläche 61. Hierdurch können sie Dehnspannungen effektiv aufnehmen.

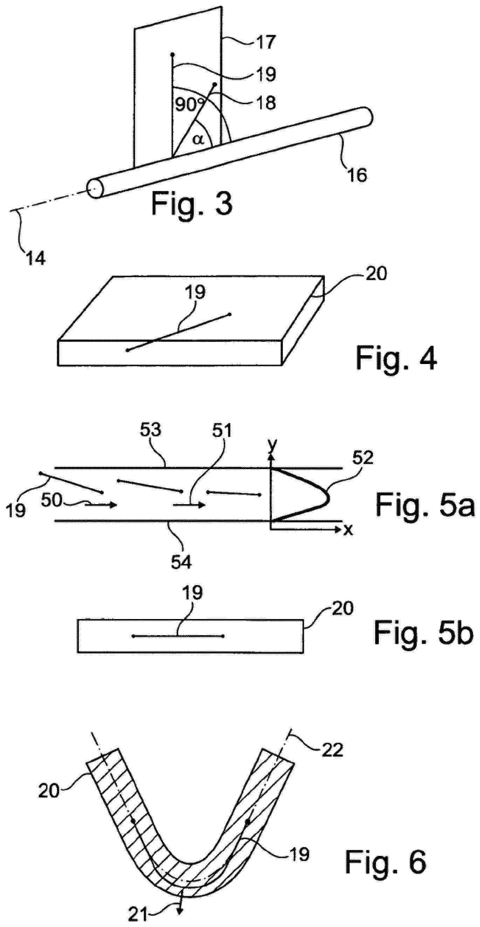

Anhand der Figur 3 soll weiter die mögliche Orientierung der Verstärkungselemente/Fasern erläutert werden. Die Rotornabe ist in Figur 3 mit 16 bezeichnet, und die Rotationsachse mit 14. Es ist eine Ebene 17 anhand eines ausgeschnittenen Rechtecks dargestellt, wobei die Ebene 17 die Rotationsachse 14 enthält, d. h., die Rotationsachse 14 verläuft vollständig in der Ebene 17. Es sei bemerkt, dass diese Darstellung vereinfacht ist, da üblicherweise die Förderelemente wendelförmig sind, so dass die Fläche 17 dann nicht mehr eben, sondern gekrümmt ist.

Es sind beispielhaft zwei Verstärkungselemente/Fasern 18, 19 eingezeichnet, die beide in der Ebene 17 im Wesentlichen radial in Bezug auf die Rotationsachse 14 verlaufen. Die Faser 18 verläuft in einem Winkel α zur Rotationsachse 14, teilweise in Axialrichtung, wobei der Winkel α vorteilhaft zwischen 45° und 90° liegt. Die Faser 19 ist derart ausgerichtet, dass sie senkrecht auf der Rotationsachse 14 steht. Ein reales Schaufelblatt ist in drei Dimensionen wendelförmig gebogen, so dass in vielen Fällen eine begrenzte Erstreckung der Verstärkungselemente/Fasern in Azimutalrichtung hinzukommt.

Die einzelnen Verstärkungselemente/Fasern müssen nicht derart positioniert sein, dass sie im Bereich der Rotationsachse 14 oder der Rotornabe 16 ihren ersten Anfangspunkt/Endpunkt haben. Sie können auch so angeordnet sein, dass sie zwischen zwei Endpunkten verlaufen, die beide von der Rotorachse 14 und/oder von der Rotornabe 16 radial beabstandet sind. Sie können sich aber auch von einem ersten radial, außen liegenden Schaufelrand bis maximal zu einem zweiten, radial gegenüber liegenden Schaufelrand, jedenfalls über die Achse hinweg, erstrecken.

In Figur 4 ist schematisch ein Ausschnitt 20 aus einem Förderelement dargestellt, wobei der Ausschnitt 20 quaderförmig gestaltet ist. In dem Abschnitt 20 ist eine Faser 19 dargestellt.

In Figur 5a wird deutlich gemacht, wie die zentrale Ausrichtung der Faser 19 erreicht wird. Das bevorzugt laminare Strömungsprofil beim Befüllen der Gießform zwischen den Begrenzungswänden 53 und 54 hat die höchste Strömungsgeschwindigkeit in der Mitte und die niedrigste Geschwindigkeit in der Nähe der Bewandung. Die nun zunächst windschief liegende Faser 19 ist in drei von links nach rechts nacheinander erreichten Winkelpositionen dargestellt und wird durch die Materialströmung, angedeutet durch die Pfeile 50, 51, infolge der Geschwindigkeitsverteilung in der Gießform in Richtung der Mitte des Strömungsprofils gezogen. Die Geschwindigkeitsverteilung der Strömung ist in dem Graphen 52 des Diagramms mit den Achsen x (≙ Geschwindigkeit) und γ (≙ Ortskoordinate in der Gießform) dargestellt.

Figur 5b zeigt eine Seitenansicht, wobei das Material des Abschnitts 20 transparent dargestellt ist, so dass der Verlauf der Faser 19 etwa in der Mitte zwischen den Begrenzungsflächen des Förderelements sichtbar ist.

Anhand der Figur 6 soll das Verhalten der Faser bei einer starken Biegung oder Knickung des Förderelements beschrieben werden.

Figur 6 zeigt den Abschnitt 20 des Förderelements nach einer Knickung. Die Faser 19 ist ebenfalls verformt. Durch eine gewisse Eigensteifigkeit der Faser kann diese jedoch wegen der Nachgiebigkeit des Materials des Förderelements beim Knicken nach außen in Richtung des Pfeils 21 ausweichen, um einen möglichst großen Krümmungsradius der Faser zu erreichen und damit einem Bruch der Faser entgegenzuwirken. Im Bereich der Knickstelle ist damit die Faser 19 aus der Mitte zwischen den Begrenzungswänden des Förderelements für die Zeitdauer der Knickung ausgelenkt. In Figur 6 ist die Mittelebene des Förderelements der Übersichtlichkeit halber wenigstens abschnittsweise durch eine gestrichelte Linie 22 dargestellt. Um diesen Effekt zu erreichen, ist eine Weichheit der Kunststoffmatrix des Rotors vorteilhaft, die einer Shorehärte < 100 D, insbesondere < 80 D, entspricht.

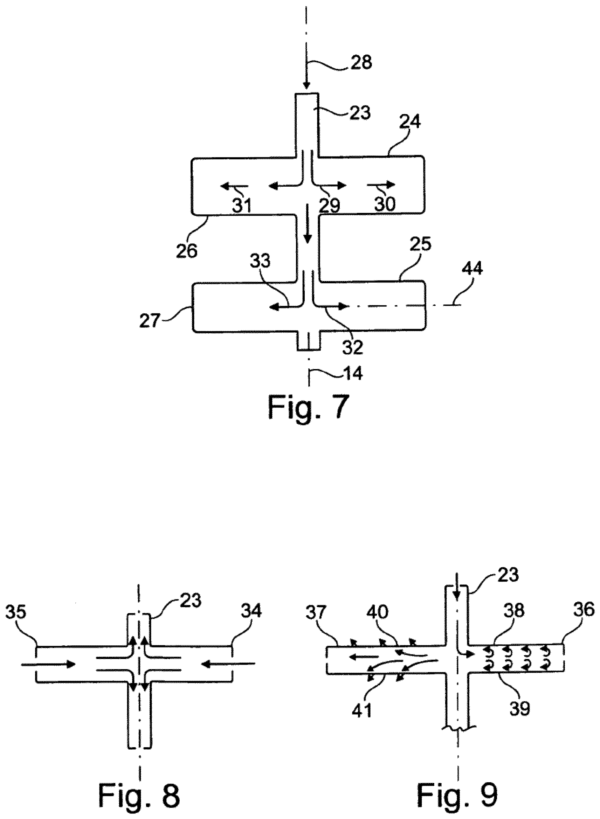

Anhand der Figur 7 soll auf die erfindungsgemäße Gestaltung eines Gusswerkzeugs / einer Gießform für einen erfindungsgemäßen Rotor eingegangen werden.

Es ist in Figur 7 ein Längsschnitt der Spritzgießform gezeigt, wobei der Bereich, der das Volumen der Rotornabe umfasst, mit 23 bezeichnet ist und die Volumina der einzelnen Förderelemente mit 24, 25, 26, 27 bezeichnet sind. In Figur 7 ist zudem eine Einspritzrichtung durch den Pfeil 28 angedeutet. Durch die weiteren Pfeile 29, 30, 31, 32, 33 ist angedeutet, dass der Spritzgusswerkstoff axial entlang der Rotationsachse 14 des entstehenden Rotors einströmt, und von dort aus radial nach außen in die Volumina der Förderelemente hinein. Beispielhaft ist die Längsachse eines der Förderelemente mit einer strichpunktierten Linie angedeutet und mit 44 bezeichnet. Werden in den Spritzgusswerkstoff entsprechend lange Verstärkungselemente/Fasern in ausreichender Anzahl eingebracht, so orientieren diese sich nach der Hauptströmungsrichtung des Werkstoffs und verbleiben beim Erstarren des Materials an Ort und Stelle.

Verdrängte Luft und überschüssiges Gießmaterial können am radial äußeren Ende der Förderelemente durch Öffnungen 45 abströmen.

In Figur 8 ist eine umgekehrte Einspritzrichtung angedeutet, wobei der Spritzgusswerkstoff von den radial äußeren Enden 34, 35 der Förderelemente radial nach innen zum Volumen des Rotors 23 eingespritzt wird. Auch in diesem Fall können lange Verstärkungselemente/Fasern mit eingebracht werden, die sich in der gemäß der Erfindung beabsichtigten Art und Weise orientieren und anordnen.

Anhand der Figur 9 wird gezeigt, dass bei schmalen Förderelementen 36, 37 die radial von innen nach außen strömende Vergussmasse an den Kanten 38, 39 des Volumens des jeweiligen Förderelements durch die Reibung verwirbelt, so dass sich keine laminare Strömung der Vergusswerkstoffs beim Einströmen ergibt.

Auf der linken Seite in der Darstellung der Figur 9 ist eine Gussformvariante dargestellt, die im Bereich der Kanten 40, 41 Überströmöffnungen oder Überströmschlitze aufweist, durch die ein Teil des Spritzgusswerkstoffs in Axialrichtung des Rotors abströmen kann, so dass die auf der rechten Seite der Figur 9 dargestellte Verwirbelung nicht entsteht. Im mittleren Bereich des Förderelements 37 ergibt sich damit eine quasi laminare Strömung, so dass die eingebrachten Verstärkungselemente/Fasern sich dort in einem Bereich um die biegetechnisch "neutrale Faser" oder neutrale Fläche (bei der im Pumpbetrieb auftretenden Biegebelastung) herum in gestreckter Form anordnen können, ohne dass sie durch Beeinflussung der Strömung des Spritzgusswerkstoffs verformt werden. Nach dem Erstarren können die Teile des Spritzgusswerkstoffs, die durch die Öffnungen an den Kanten 40, 41 der Volumina der Förderelemente in der Spritzgießform ausgetreten sind, entfernt werden, beispielsweise durch Abschneiden. Gleiches kann, wie in Figur 7, an den Außenkanten der Förderelemente erfolgen. Hier sind zwei Überströmkanäle 45 vorgesehen, durch die der Kunststoff ohne Verstärkungselemente/Fasern entweichen kann.

Durch eine asymmetrische Verteilung der Abströmöffnungen, beispielsweise einen größeren Abströmquerschnitt in der Nähe des Bereiches des Förderelements, das im Betrieb dem Fluidgegendruck der Flüssigkeit ausgesetzt ist, ergibt sich dort eine stärkere Strömung des Gießwerkstoffs, so dass dort mehr Verstärkungselemente/Fasern als auf der der "neutralen Fläche" gegenüberliegenden Seite eingelagert werden, mit dem Ergebnis, dass die Dehnspannungen durch eine hohe Anzahl von Verstärkungselementen/Fasern aufgenommen werden können.

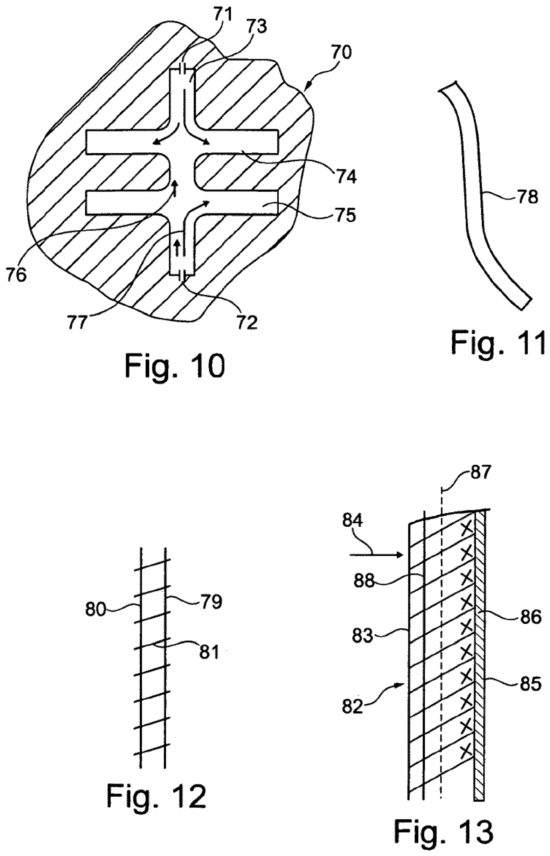

In Figur 10 ist schematisch eine Spritzgießform 70 mit einer ersten Einspritzöffnung 71 und einer zweiten Einspritzöffnung 72 dargestellt, wobei die beiden Einspritzöffnungen 71, 72 an einander gegenüberliegenden Enden des zentralen Hohlraums 73 angeordnet sind, in dem durch den Verguss die Nabe des Rotors gebildet wird. Die Hohlräume, in denen die Förderelemente entstehen, sind nur sehr schematisch angedeutet und mit 74, 75 bezeichnet. Die Pfeile 76, 77 bezeichnen die Strömungsrichtungen des in die Gussform einströmenden Kunststoffs. Wird der Kunststoff nacheinander oder in wechselnden Mengenverhältnissen durch jeweils die erste Einspritzöffnung 71 und die zweite Einspritzöffnung 72 in die Form eingespritzt, so ergeben sich verschiedene Strömungsrichtungen des flüssigen Gießwerkstoffs. Wenn diesem die Verstärkungselemente hinzugefügt werden, so ergeben sich entsprechend auch unterschiedliche Ausrichtungen der Verstärkungselemente in dem entstehenden Rotor. Auf diese Weise können unterschiedliche Ausrichtungsmuster durch Steuerung der Einspritzgeschwindigkeit durch die Einspritzöffnungen und Änderung der Einspritzgeschwindigkeiten bzw. des Verhältnisses der Einspritzgeschwindigkeiten durch die verschiedenen Einspritzöffnungen während des Gießverfahrens gestaltet werden.

In Figur 11 ist in einer perspektivischen Ansicht ein Verstärkungselement in Form einer Metallfolie oder einer Kunststofffolie 78 dargestellt, die beispielsweise nur wenige Mikrometer dick, wenige Zehntelmillimeter breit und wenige Millimeter lang sein kann.

Figur 12 zeigt als Verstärkungselement einen länglichen Gewebestreifen, der schematisch nur zwei in Längsrichtung verlaufende Fasern 79, 80 und eine Vielzahl von quer zu diesen verlaufenden kürzeren Fasern 81 aufweist. Ein solches Gewebe kann in den beiden Längsrichtungen der vorhandenen Fasern besonders gut Zugkräfte aufnehmen.

In Figur 13 ist im Querschnitt schematisch ein Bereich eines Förderelementes 82 dargestellt. Dabei ist mit 83 die Oberfläche bezeichnet, die im Betrieb auf der Druckseite liegt und die dem Fluidgegendruck, angedeutet durch den Pfeil 84, ausgesetzt ist.

Die dieser Seite oder Deckfläche 83 gegenüberliegende Deckfläche ist mit 85 bezeichnet. In dem darstellten Beispiel ist auf dieser Seite des Förderelementes 82 eine Beschichtung 86 vorgesehen, die durch eine aufgeklebte Folie oder eine flüssige Beschichtung, beispielsweise Lackierung, gegeben sein kann.

Die bei der Biegung des Förderelementes 82 im Pumpbetrieb im Sinne der Mechanik neutrale "Faser" oder Fläche ist gestrichelt eingezeichnet und mit 87 bezeichnet.

Wird angenommen, dass das Förderelement, wie in Figur 13 dargestellt, sich im kräftefreien Zustand befindet, d. h., dass auf das Förderelement keine äußeren Kräfte wirken, so kann vorgesehen sein, dass in diesem Zustand die Verstärkungselemente, von denen eines beispielhaft eingezeichnet und mit 88 bezeichnet ist, gestreckt und kraftfrei vorliegen,

Wird dann durch die Wirkung des Fluidgegendrucks 84 bei Rotation des Rotors In einem Fluid eine Biegekraft auf das Förderelement in Richtung des Pfeils 84 gebracht, so werden die Verstärkungselemente 88 auf Zug beansprucht, da auf der in der Figur linken Seite der neutralen Faser 87 infolge der Biegung des Förderelementes eine Verlängerung auftritt. Dieser Biegung wird durch die Verstärkungselemente 88 eine Grenze gesetzt, da diese praktisch dehnfest sind.

Um den Formunterschied des Rotors zwischen dem zweiten, kraftfreien Zustand und einem dritten Zustand, dem Belastungszustand, weiter zu verringern, kann vorgesehen sein, dass im kraftfreien Zustand, d. h. ohne Einwirkung von äußeren Kräften auf den Rotor, die Verstärkungselemente 88 bereits durch innere Materialspannung vorgespannt sind. Dies gelingt dadurch, dass nach der Herstellung des Rotors, genauer nach Vollendung des Spritzgießvorgangs, das Förderelement entweder auf der Seite der Deckfläche 83 gedehnt oder auf der Seite der Deckfläche 85 geschrumpft wird.

Dies gelingt beispielsweise dadurch, dass auf die Deckfläche 85 nach dem Spritzgießvorgang oder während des Spritzgießvorgangs, beispielsweise durch Imprägnieren der Spritzgießform, eine Beschichtung 86 aufgebracht wird, die während des Trocknens oder Vernetzens oder durch eine nachfolgende Behandlung, insbesondere Strahlenvernetzung, UV-Vernetzung oder thermische Behandlung, geschrumpft werden kann. Die Strahlenvernetzung kann beispielsweise auch mittels eines oder mehrerer Laserstahlen eingebracht und damit lokal sehr fokussiert appliziert werden.

Es ist jedoch auch denkbar, dass zusätzlich oder alternativ zu einer Deckschicht 86 das Material des Förderelementes 82 auf der Seite der neutralen Fläche der Faser 87, die der Deckschicht 85 zugewandt ist, geschrumpft wird, beispielsweise durch eine Wärmebehandlung oder durch eine Vernetzung/ Polymerisation, die auf der anderen Seite des Förderelementes nicht oder nur in geringerem Maße stattfindet.

In den Figuren 14a und 14b ist ein beispielhaft ein Gewebe aus Fasern dargestellt. Die Faser 90 verläuft dabei als Teil eines Faserbündels in einem Winkel zu weiteren Faserbündeln 91, wobei die Faserbündel 91 senkrecht zur Zeichenfläche angeordnet sind und von der Faser 90 abwechselnd links- bzw. rechtsseitig passiert werden. In Fig. 14a, welche den entspannten Zustand des Rotors repräsentiert, haben die Faserbündel 91 einen Abstand a zueinander und die Faser 90 hat einen hin- und her gekrümmten Verlauf. In Fig. 14b, welche den Zustand des Rotors im Betrieb repräsentiert, haben die Faserbündel 91 einen größeren Abstand a' zueinander und die Faser 90 hat einen weniger gekrümmten Verlauf. Aus den Figuren 14a und 14b wird ersichtlich, dass sich ein solches System bereits wesentlich stabilisiert, bevor eine vollständige Streckung der Faser 90 erreicht ist, da über den Zustand der Fig. 14b hinaus die Faserbündel 91 sich rechtwinklig zur Faser 90 im Grundwerkstoff bewegen müssten, was durch diesen und die Faser 90 im Wesentlichen verhindert wird.

Fig. 15a zeigt einen Rotor in einem expandierten kraftfreien Zustand in einer perspektivischen Ansicht und die Kennzeichnung einer Schnittebene A-A. Fig. 15b zeigt denselben Rotor in ebendieser Schnittansicht. Die gestrichelten Linien 101 und 102 repräsentieren die jeweils biegeneutrale Faser bzw. Fläche des jeweiligen Schaufelblatts. Die Fasern 103 und 104 zeigen beispielhaft den Verlauf von zwei Fasern in der dargestellten Schnittebene. Die Fasern sind in diesem Zustand gekrümmt. Im Betriebszustand unter Last wirkt durch das Fluid ein Druck in Richtung der dargestellten Pfeile auf die Schaufelblätter, wodurch diese sich weiter aufstellen. Bei voller Betriebslast sind die Fasern 103 und 104 zumindest in den mit 105 bzw. 106 gekennzeichneten Bereichen gestreckt, wodurch eine weiteres Aufstellen der Schaufelblätter behindert wird, da die sehr zugsteifen Fasern, dann in ihrer axialen Ausdehnung gestreckt werden müssten. Der Vorteil einer solchen Auslegung ist, dass die Form des Schaufelblattes oberhalb einer Mindestdrehzahl nahezu konstant ist.

Fig. 16a zeigt einen erfindungsgemäßen Rotor in einem expandierten kraftfreien Zustand in einer perspektivischen Ansicht und die Kennzeichnung einer Schnittebene B-B. Fig. 16b zeigt denselben Rotor in ebendieser Schnittansicht. Die gestrichelten Linien 111 und 112 repräsentieren die jeweils biegeneutrale Faser bzw. Fläche des jeweiligen Schaufelblatts. Die Fasern 113 und 114 zeigen beispielhaft den Verlauf von zwei Fasern in der dargestellten Schnittebene. Wie man erkennen kann, sind die Fasern in diesem Beispiel bereits in einem weiten Bereich gestreckt. Da die Fasern sehr zugsteif sind, ist eine weitere Dehnung der Fasern kaum möglich. Auch unter den im Betriebszustand auftretenden Kräften, welche durch den Strömungsdruck auf die Schaufelblätter einwirken und welche in der Fig. 16b durch Pfeile dargestellt sind, wird sich ein solches Schaufelblatt nicht mehr wesentlich verformen, mit dem Vorteil dass die Schaufelblattform über nahezu den gesamten Drehzahlbereich hinweg nahezu unverändert ist.

Fig. 17a zeigt einen erfindungsgemäßen Rotor in einem expandierten kraftfreien Zustand in einer seitlichen Ansicht mit der Darstellung einer Schnitteben C-C. Fig. 17b zeigt denselben Rotor in ebendieser Schnittansicht. Die gestrichelten Linien 121 und 122 repräsentieren die jeweils biegeneutrale Faser bzw. Fläche des jeweiligen Schaufelblatts. Die Fasern 123 und 124 zeigen beispielhaft den Verlauf von zwei Fasern in der dargestellten Schnittebene. Wie man erkennen kann, sind die Fasern in diesem Beispiel bereits in einem weiten Bereich gestreckt. Da die Fasern sehr zugsteif sind, ist eine weitere Dehnung der Fasern kaum möglich. Auch unter den im Betriebszustand auftretenden Kräften, welche durch den Strömungsdruck auf die Schaufelblätter einwirken und welche in der Fig. 17b durch Pfeile dargestellt sind, wird sich ein solches Schaufelblatt nicht mehr wesentlich verformen.

Durch die oben angegebenen Merkmale, insbesondere durch Gestaltung des Rotors und Einbringen geeigneter Verstärkungselemente/Fasern, wird eine stabile Gestaltung eines Rotors mit ausreichender Formtreue auch nach teilweiser Überstreckung oder häufiger Wechselbelastung oder konstant anliegender Biegebelastung erreicht. Durch die erläuterten Herstellungsverfahren und die dargestellte Spritzgießform wird eine sinnvolle und vorteilhafte Herstellungsmöglichkeit für den vorgestellten Rotor aufgezeigt.

Die Erfindung wird durch die Ansprüche definiert. Teil der Offenbarung bilden zudem folgende Aspekte, die zur Erläuterung der Erfindung dienen können.
1. Aspekt: Rotor für eine komprimierbare Fluidpumpe, insbesondere eine durch ein Blutgefäß in einen Patientenkörper einführbare Blutpumpe, der ein oder mehrere Förderelemente (15) aufweist und radial zwischen einem ersten komprimierten und einem zweiten, radial expandierten und von äußeren Kräften freien Zustand komprimierbar und expandierbar ist und der teilweise aus einem durch strangförmige Verstärkungselemente, insbesondere Fasern, verstärkten Kunststoff besteht und zur Rotation um eine Rotationsachse vorgesehen ist, wobei der Rotor im ersten, komprimierten Zustand gespannt und im zweiten, expandierten Zustand frei von äußeren Spannungen ist und wobei ein dritter Zustand existiert, den der Rotor (42) im Betriebszustand unter Last einnimmt, wobei verschiedene Materialien des Rotors und ihre Verteilung derart aufeinander abgestimmt sind, dass im zweiten Zustand des Rotors gezielt Materialspannungen erzeugt sind, die die Verstärkungselemente auf Zug beanspruchen und/oder strecken.
2. Aspekt: Rotor nach Aspekt 1, wobei zudem vorgesehen ist, dass die Verstärkungselemente von dem Kunststoff, aus dem der Rotor überwiegend besteht, wenigstens auf einem Anteil von 90 %, insbesondere 99 %, ihrer Oberfläche, weiter insbesondere vollständig, umschlossen sind.
3. Aspekt: Rotor nach Aspekt 1 oder 2, wobei zudem vorgesehen ist, dass das Kunststoffmaterial, das die Verstärkungselemente einbettet, auf der im Betrieb von dem Fluidgegendruck nicht belasteten Seite der Förderelemente, insbesondere auf der entsprechenden Seite des Förderelementes bezüglich der bei der Biegebelastung im Pumpbetrieb biegeneutralen Faser oder Fläche, wenigstens bereichsweise andere Eigenschaften aufweist als auf der mit dem Fluidgegendruck belasteten Seite der Förderelemente, insbesondere auf der von dem Fluidgegendruck nicht belasteten Seite stärker vernetzt oder geschrumpft ist oder dort auf der Oberfläche eine auf dem Förderelement geschrumpfte Auflage in Form von einer oder mehreren Folien, Beschichtungen oder Fasern trägt.
4. Aspekt: Rotor nach Aspekt 1, 2 oder 3, wobei zudem vorgesehen ist, dass ein oder mehrere Förderelemente des Rotors durch Spritzgießen mit gleichzeitigem Hinzufügen von Verstärkungselementen im expandierten Zustand hergestellt ist, wobei die Verstärkungselemente durch einen Spritzgießwerkstoff allseitig umschlossen sind und wenigstens abschnittsweise im expandierten Zustand gestreckt, insbesondere zu wenigstens 90 %, weiter insbesondere zu 95 %, weiter insbesondere zu 99 % gestreckt vorliegen.
5. Aspekt: Rotor nach Aspekt 1, 2, 3 oder 4, bei dem zudem vorgesehen ist, dass die Verstärkungselemente im zweiten, expandierten Zustand des Rotors ohne einen Fluidgegendruck in einem solchen Maß gestreckt vorliegen, dass sie beim Übergang zu einem dritten Zustand, der den Betriebszustand mit einem Fluidgegendruck darstellt, um weniger als 5 %, insbesondere weniger als 1 %, verlängern, wobei die Verlängerung insbesondere am Abstand der beiden Enden eines Verstärkungselementes voneinander bemessen ist.
6. Aspekt: Rotor nach Aspekt 1, 2, 3, 4 oder 5, wobei zudem vorgesehen ist, dass in einem zweiten, expandierten Zustand des Rotors und/oder einem dritten Betriebszustand mit Fluidgegendruck wenigstens ein Anteil der Verstärkungselemente, insbesondere mindestens 10%, weiter insbesondere mindestens 30%, in wenigstens einem Bereich eines Förderelementes, in dem dieses gekrümmt ist, gestreckt und gerade verlaufen.
7. Aspekt: Verfahren zum Herstellen eines Rotors für eine Fluidpumpe, insbesondere nach dem ersten Aspekt, wobei vorgesehen ist, dass der Rotor nach dem Spritzgießen einer Behandlung unterworfen wird, die eine unterschiedliche Schrumpfung und/oder Vernetzung des Gussmaterials auf der im Betrieb durch den Fluidgegendruck belasteten Seite der Förderelemente als auf der dieser gegenüberliegenden Seite bewirkt.
8. Aspekt: Verfahren zum Herstellen eines Rotors für eine Fluidpumpe, insbesondere nach dem ersten Aspekt, durch Spritzgießen, wobei vorgesehen ist, dass auf der der im Betrieb einem Fluidgegendruck ausgesetzten Seite der Förderelemente gegenüberliegenden Seite auf wenigstens eines der Förderelemente eine schrumpfbare Schicht aufgebracht wird.
9. Aspekt: Gießform für einen Rotor für eine Fluidpumpe mit Förderelementen nach dem ersten Aspekt, bei der wenigstens zwei verschiedene EinspritzÖffnungen vorgesehen sind.
10. Aspekt: Rotor nach einem der vorhergehenden Aspekte, wobei vorgesehen ist, dass die Verstärkungselemente zweidimensionale Ausdehnung haben, beispielsweise als Folienstücke oder Gewebe mit Gruppen von einander kreuzenden Fasern (siehe Figuren 14a und 14b) ausgeführt sind.

Die Erfindung wird durch die Ansprüche definiert. Teil der Offenbarung bilden zudem folgende Aspekte, die zur Erläuterung der Erfindung dienen können.
1. Rotor für eine komprimierbare Fluidpumpe, insbesondere eine durch ein Blutgefäß in einen Patientenkörper einführbare Blutpumpe, der ein oder mehrere Förderelemente (15) aufweist und radial zwischen einem ersten, komprimierten und einem zweiten, radial expandierten Zustand komprimierbar und expandierbar ist und der wenigstens teilweise aus einem durch strangförmige Verstärkungselemente, insbesondere Fasern (10, 11, 13, 18, 19, 55, 56, 62, 63) verstärkten Kunststoff besteht und zur Rotation um eine Rotationsachse (14) vorgesehen ist, wobei der Rotor im ersten, komprimierten Zustand gespannt und im zweiten, expandierten Zustand frei von äußeren Spannungen ist und wobei ein dritter Zustand existiert, den der Rotor (42) im Betriebszustand unter Last einnimmt, dadurch gekennzeichnet, dass die Fasern in dem Rotor im dritten Zustand wenigstens abschnittsweise gestreckt verlaufen.
2. Rotor nach Aspekt 1, dadurch gekennzeichnet, dass die Verstärkungselemente, insbesondere Fasern (10, 11, 13, 18, 19, 55, 56, 62, 63) im dritten Zustand (Betriebszustand) des Rotors wenigstens abschnittsweise in Bereichen des Rotors (42), in denen dieser eine Dehnspannung aufweist, gestreckt und im Wesentlichen in Richtung der Dehnspannung verlaufen.
3. Rotor nach Aspekt 1 oder 2, dadurch gekennzeichnet, dass mehr als die Hälfte der Verstärkungselemente, insbesondere Fasern (10, 11, 13, 18, 19, 55, 56, 62, 63) in Bereichen des Rotors und/oder der Förderelemente angeordnet sind, in denen dieser/diese im Betriebszustand Dehnspannungen aufweist/aufweisen.
4. Rotor nach Aspekt 1, 2 oder 3, dadurch gekennzeichnet, dass bei dem Rotor (42) zwischen dem zweiten, expandierten Zustand in dem dritten Betriebszustand bezüglich der äußeren Form im Wesentlichen keine Unterschiede bestehen.
5. Rotor nach Aspekt 1, 2, 3 oder 4, dadurch gekennzeichnet, dass Verstärkungselemente, insbesondere Fasern (10, 11, 13, 18, 19, 55, 56, 62, 63) vorgesehen sind, die sich in radialer Richtung über die Rotationsachse (14) hinaus erstrecken.
6. Rotor nach Aspekt 1, 2, 3, 4 oder 5, dadurch gekennzeichnet, dass im Betriebszustand wenigstens ein Teil der Verstärkungselemente, insbesondere Fasern, insbesondere die Mehrheit der Verstärkungselemente/Fasern (10, 11, 13, 18, 19, 55, 56, 62, 63), abschnittsweise gerade verlaufen.
7. Rotor nach Aspekt 1, 2, 3, 4 oder 5, dadurch gekennzeichnet, dass im Betriebszustand wenigstens ein Teil der Verstärkungselemente, insbesondere Fasern (10, 11, 13, 18, 19, 55, 56, 62, 63), insbesondere die Mehrheit der Verstärkungselemente/Fasern, entlang der Längsrichtung des jeweiligen Förderelementes (15) mit einer geringeren Krümmung verlaufen als die neutrale Faser (Nulllinie) und/oder neutrale Fläche des jeweiligen Förderelementes im Sinne der Festigkeitslehre.
8. Rotor nach Aspekt 1 oder einem der folgenden, dadurch gekennzeichnet, dass der Kunststoff des Rotors (42) eine Shore-Härte <100 D, insbesondere < 80 D, aufweist.
9. Rotor nach Aspekt 1 oder einem der folgenden, dadurch gekennzeichnet, dass ein erster Anteil von mehr als 30%, insbesondere mehr als 50%, der Verstärkungselemente/Fasern (10, 11, 13, 18, 19, 55, 56, 62, 63) im expandierten Zustand des Rotors (42) im Wesentlichen gestreckt von einem der Rotorachse (14) am nächsten liegenden Abschnitt (10a, 11a, 13a) zu einem der Rotationsachse (14) ferner liegenden zweiten Abschnitt (10b, 11b, 13b) verläuft.
10. Rotor nach Aspekt 1 oder einem der folgenden, dadurch gekennzeichnet, dass ein erster Anteil der Verstärkungselemente/Fasern (10, 11, 13, 18, 19, 55, 56, 62, 63), der mehr als 30% der Verstärkungselemente/Fasern, insbesondere mehr als 50%, der Verstärkungselemente/Fasern beträgt, eine Länge aufweist, die mindestens 30%, insbesondere mindestens 50%, der Maximalhöhe der Förderelemente (15), gemessen in Radialrichtung des Rotors (42) im expandierten Zustand, entspricht.
11. Rotor nach Aspekt 9 oder 10, dadurch gekennzeichnet, dass der erste Anteil der Verstärkungselemente/Fasern (10, 11, 13, 18, 19, 55, 56, 62, 63) im Wesentlichen senkrecht zur Rotationsachse (14) verläuft.
12. Rotor nach Aspekt 1 oder einem der folgenden, dadurch gekennzeichnet, dass der Durchmesser der Verstärkungselemente/Fasern (10, 11, 13, 18, 19, 55, 56, 62, 63), insbesondere des ersten Anteils der Verstärkungselemente/Fasern (10, 11, 13, 18, 19, 55, 56, 62, 63), kleiner als 40 Mikrometer ist.
13. Rotor nach Aspekt 1 oder einem der folgenden, dadurch gekennzeichnet, dass die Oberfläche der Verstärkungselemente/Fasern (10, 11, 13, 18, 19, 55, 56, 62, 63) mit einem Haftvermittler versehen ist.
14. Rotor nach Aspekt 1 oder einem der folgenden, dadurch gekennzeichnet, dass ein Anteil von Verstärkungselementen/Fasern im expandierten Zustand des Rotors zu den Verstärkungselementen/Fasern des ersten Teils quer verläuft und insbesondere mit diesen im Mittel Winkel von mindestens 30° einschließt.
15. Rotor nach Aspekt 1 oder einem der folgenden, dadurch gekennzeichnet, dass die Verstärkungselemente/Fasern wenigstens teilweise in Form von Gewebeabschnitten mit längs und quer verlaufenden Fasern vorliegen.
16. Rotor nach Aspekt 1 oder einem der folgenden, dadurch gekennzeichnet, dass die Verstärkungselemente in Form von Folienstreifen vorliegen, deren Länge wenigstens dreimal, insbesondere wenigstens fünfmal, weiter insbesondere wenigstens zehnmal so groß ist wie ihre Breite.
17. Rotor nach Aspekt 1 oder einem der folgenden, dadurch gekennzeichnet, dass die Verstärkungselemente von dem Kunststoff, aus dem der Rotor überwiegend besteht, wenigstens auf einem Anteil von 90%, insbesondere 99 %, ihrer Oberfläche, weiter insbesondere vollständig, umschlossen sind.
18. Rotor nach Aspekt 1 oder einem der folgenden, dadurch gekennzeichnet, dass das Kunststoffmaterial, das die Verstärkungselemente einbettet, auf der im Betrieb von dem Fluidgegendruck nicht belasteten Seite der Förderelemente wenigstens bereichsweise andere Eigenschaften aufweist als auf der mit dem Fluidgegendruck belasteten Seite der Förderelemente, insbesondere auf der von dem Fluidgegendruck nicht belasteten Seite stärker vernetzt oder geschrumpft ist oder dort auf der Oberfläche eine auf dem Förderelement (82) geschrumpfte Auflage (86) in Form von einer oder mehreren Folien, Beschichtungen oder Fasern trägt.
19. Rotor für eine komprimierbare Fluidpumpe, der radial zwischen einem ersten, komprimierten und einem zweiten, expandierten Zustand expandierbar und komprimierbar ist, dadurch gekennzeichnet, dass ein oder mehrere Förderelemente des Rotors durch Spritzgießen mit gleichzeitigem Hinzufügen von Verstärkungselementen im expandierten Zustand hergestellt ist, wobei die Verstärkungselemente durch einen Spritzgießwerkstoff allseitig umschlossen sind und wenigstens abschnittsweise im expandierten Zustand gestreckt, insbesondere zu wenigstens 90 %, weiter insbesondere zu 95 %, weiter insbesondere zu 99 % gestreckt vorliegen.
20. Rotor nach Aspekt 1 oder einem der folgenden, dadurch gekennzeichnet, dass die Verstärkungselemente im zweiten, expandierten Zustand des Rotors ohne einen Fluidgegendruck in einem solchen Maß gestreckt vorliegen, dass sie sich beim Übergang zu einem dritten Zustand, der den Betriebszustand mit einem Fluidgegendruck darstellt, um weniger als 5%, insbesondere weniger als 1 %, verlängern, wobei die Verlängerung insbesondere am Abstand der beiden Enden eines Verstärkungselementes voneinander bemessen ist.
21. Rotor nach Aspekt 1 oder einem der folgenden, dadurch gekennzeichnet, dass in einem zweiten, expandierten Zustand des Rotors und/oder einem dritten Betriebszustand mit Fluidgegendruck wenigstens ein Anteil der Verstärkungselemente, insbesondere mindestens 10 %, weiter insbesondere mindestens 30%, in wenigstens einem Bereich eines Förderelementes, in dem dieses gekrümmt ist, gestreckt und gerade verlaufen.
22. Rotor nach Aspekt 21, dadurch gekennzeichnet, dass in einem Bereich eines Förderelementes, in dem dieses gekrümmt ist, wenigstens zwei Anteile von Verstärkungselementen gerade und gestreckt verlaufen, wobei die Richtungen, in denen die Verstärkungselemente der Anteile verlaufen, bei dem jeweiligen Anteil parallel, jedoch bei den verschiedenen Anteilen verschieden sind.
23. Rotor nach Aspekt 1 oder einem der folgenden, dadurch gekennzeichnet, dass die Länge der Verstärkungselemente bei wenigstens 30%, insbesondere wenigstens 50%, der Elemente größer ist als die durchschnittliche Dicke der Förderelemente, insbesondere wenigstens zweimal so lang, weiter insbesondere wenigstens 5-mal oder 10-mal so lang.
24. Rotor nach Aspekt 1 oder einem der folgenden, dadurch gekennzeichnet, dass Verstärkungselemente, insbesondere Fasern, in den sie einbettenden Kunststoff in einem Spritzgussverfahren eingebracht sind und während der Zeit, in der sich der Rotor in der Spritzgussform befindet, einen abschnittsweise gekrümmten Verlauf entlang der Strömung des Kunststoffs in die Spritzgussform aufweisen.
25. Verfahren zur Herstellung eines Rotors (42) nach Aspekt 1 oder einem der folgenden mittels eines Gießverfahrens, insbesondere eines Spritzgießverfahrens, bei dem das Material der Förderelemente (15) in Radialrichtung in Bezug auf die Rotorachse (14) in die Volumina der Förderelemente derart eingebracht wird, dass der Gießwerkstoff in das Volumen jedes einzelnen Förderelementes jeweils in Radialrichtung (30, 31, 32, 33) einströmt.
26. Verfahren zur Herstellung eines Rotors nach einem der Aspekte 1 bis 24, dadurch gekennzeichnet, dass der Rotor durch Gießen, insbesondere Spritzgießen, hergestellt wird, wobei das Spritzgießverfahren in zwei aufeinanderfolgenden Phasen mit unterschiedlichen Einspritzrichtungen und/oder von zwei verschiedenen Einspritzstellen aus durchgeführt wird.
27. Verfahren nach Aspekt 25 oder 26, dadurch gekennzeichnet, dass der Rotor nach dem Spritzgießen einer Behandlung unterworfen wird, die eine unterschiedliche Schrumpfung und/oder Vernetzung des Gussmaterials auf der im Betrieb durch den Fluidgegendruck belasteten Seite der Förderelemente als auf der dieser gegenüberliegenden Seite bewirkt.
28. Verfahren nach Aspekt 25, 26 oder 27, dadurch gekennzeichnet, dass auf der der im Betrieb einem Fluidgegendruck ausgesetzten Seite der Förderelemente gegenüberliegenden Seite auf wenigstens eines der Förderelemente eine schrumpfbare Schicht aufgebracht wird.
29. Gießform für einen Rotor (42) gemäß einem der Aspekte 1 bis 24, dadurch gekennzeichnet, dass bei den Volumina der Förderelemente (15) an deren radial verlaufenden Kanten (40, 41) Überströmkanäle vorgesehen sind, um einen störungsfreien Fluss des Gießwerkstoffs in Radialrichtung zu ermöglichen.
30. Gießform für einen Rotor gemäß einem der Aspekte 1 bis 24, gekennzeichnet durch wenigstens zwei verschiedene Einspritzöffnungen.

## Patentansprüche

1. Rotor für eine komprimierbare Fluidpumpe, insbesondere eine durch ein Blutgefäß in einen Patientenkörper einführbare Blutpumpe, der ein oder mehrere Förderelemente (15) aufweist und radial zwischen einem ersten, komprimierten und einem zweiten, radial expandierten Zustand komprimierbar und expandierbar ist und der wenigstens teilweise aus einem durch strangförmige Verstärkungselemente, nämlich Fasern (10, 11, 13, 18, 19, 55, 56, 62, 63) verstärkten Kunststoff besteht und zur Rotation um eine Rotationsachse (14) vorgesehen ist, wobei der Rotor im ersten, komprimierten Zustand gespannt und im zweiten, expandierten Zustand frei von äußeren Spannungen ist und wobei ein dritter Zustand existiert, den der Rotor (42) im Betriebszustand unter Last einnimmt, **dadurch gekennzeichnet, dass** die Fasern von dem Kunststoff, aus dem der Rotor überwiegend besteht, wenigstens auf einem Anteil von 90 % ihrer Oberfläche umschlossen sind, wobei die Fasern in dem Rotor im dritten Zustand wenigstens abschnittsweise gestreckt verlaufen, wobei in dem zweiten, expandierten Zustand des Rotors wenigstens ein Anteil der Verstärkungselemente in wenigstens einem Bereich des Förderelementes, in dem dieses gekrümmt ist, bereits gestreckt und gerade verläuft.

2. Rotor nach Anspruch 1, **dadurch gekennzeichnet, dass** die Verstärkungselemente, insbesondere Fasern (10, 11, 13, 18, 19, 55, 56, 62, 63) im dritten Zustand (Betriebszustand) des Rotors wenigstens abschnittsweise in Bereichen des Rotors (42), in denen dieser eine Dehnspannung aufweist, gestreckt und im Wesentlichen in Richtung der Dehnspannung verlaufen.

3. Rotor nach Anspruch 1 oder 2, **dadurch gekennzeichnet, dass** mehr als die Hälfte der Verstärkungselemente, insbesondere Fasern (10, 11, 13, 18, 19, 55, 56, 62, 63) in Bereichen des Rotors und/oder der Förderelemente angeordnet sind, in denen dieser/diese im Betriebszustand Dehnspannungen aufweist/aufweisen.

4. Rotor nach Anspruch 1, 2 oder 3, **dadurch gekennzeichnet, dass** bei dem Rotor (42) zwischen dem zweiten, expandierten Zustand in dem dritten Betriebszustand bezüglich der äußeren Form im Wesentlichen keine Unterschiede bestehen.

5. Rotor nach einem der Ansprüche 1 bis 4, **dadurch gekennzeichnet, dass** im Betriebszustand wenigstens ein Teil der Verstärkungselemente, insbesondere Fasern, insbesondere die Mehrheit der Verstärkungselemente/Fasern (10, 11, 13, 18, 19, 55, 56, 62, 63), abschnittsweise gerade verlaufen.

6. Rotor nach einem der Ansprüche 1 bis 4, **dadurch gekennzeichnet, dass** im Betriebszustand wenigstens ein Teil der Verstärkungselemente, insbesondere Fasern (10, 11, 13, 18, 19, 55, 56, 62, 63), insbesondere die Mehrheit der Verstärkungselemente/Fasern, entlang der Längsrichtung des jeweiligen Förderelementes (15) mit einer geringeren Krümmung verlaufen als die neutrale Faser (Nulllinie) und/oder neutrale Fläche des jeweiligen Förderelementes im Sinne der Festigkeitslehre.

7. Rotor nach Anspruch 1 oder einem der folgenden, **dadurch gekennzeichnet, dass** der Kunststoff des Rotors (42) eine Shore-Härte <100 D, insbesondere < 80 D, aufweist.

8. Rotor nach Anspruch 1 oder einem der folgenden, **dadurch gekennzeichnet, dass** ein erster Anteil von mehr als 30%, insbesondere mehr als 50%, der Verstärkungselemente/Fasern (10, 11, 13, 18, 19, 55, 56, 62, 63) im expandierten Zustand des Rotors (42) im Wesentlichen gestreckt von einem der Rotorachse (14) am nächsten liegenden Abschnitt (10a, 11a, 13a) zu einem der Rotationsachse (14) ferner liegenden zweiten Abschnitt (10b, 11b, 13b) verläuft.

9. Rotor nach Anspruch 1 oder einem der folgenden, **dadurch gekennzeichnet, dass** ein erster Anteil der Verstärkungselemente/Fasern (10, 11, 13, 18, 19, 55, 56, 62, 63), der mehr als 30 % der Verstärkungselemente/Fasern, insbesondere mehr als 50%, der Verstärkungselemente/Fasern beträgt, eine Länge aufweist, die mindestens 30%, insbesondere mindestens 50%, der Maximalhöhe der Förderelemente (15), gemessen in Radialrichtung des Rotors (42) im expandierten Zustand, entspricht.

10. Rotor nach Anspruch 8 oder 9, **dadurch gekennzeichnet, dass** der erste Anteil der Verstärkungselemente/Fasern (10, 11, 13, 18, 19, 55, 56, 62, 63) im Wesentlichen senkrecht zur Rotationsachse (14) verläuft.

11. Rotor nach Anspruch 1 oder einem der folgenden, **dadurch gekennzeichnet, dass** der Durchmesser der Verstärkungselemente/Fasern (10, 11, 13, 18, 19, 55, 56, 62, 63), insbesondere des ersten Anteils der Verstärkungselemente/Fasern (10, 11, 13, 18, 19, 55, 56, 62, 63), kleiner als 40 Mikrometer ist, und/oder **dadurch gekennzeichnet, dass** die Oberfläche der Verstärkungselemente/Fasern (10, 11, 13, 18, 19, 55, 56, 62, 63) mit einem Haftvermittler versehen ist, und/oder **dadurch gekennzeichnet, dass** ein Anteil von Verstärkungselementen/Fasern im expandierten Zustand des Rotors zu den Verstärkungselementen/Fasern des ersten Teils quer verläuft und insbesondere mit diesen im Mittel Winkel von mindestens 30° einschließt, und/oder **dadurch gekennzeichnet, dass** die Verstärkungselemente/Fasern wenigstens teilweise in Form von Gewebeabschnitten mit längs und quer verlaufenden Fasern vorliegen, und/oder **dadurch gekennzeichnet, dass** die Verstärkungselemente in Form von Folienstreifen vorliegen, deren Länge wenigstens dreimal, insbesondere wenigstens fünfmal, weiter insbesondere wenigstens zehnmal so groß ist wie ihre Breite.

12. Rotor nach Anspruch 1 oder einem der folgenden, **dadurch gekennzeichnet, dass** die Verstärkungselemente von dem Kunststoff, aus dem der Rotor überwiegend besteht, wenigstens auf einem Anteil von 99 % ihrer Oberfläche, weiter insbesondere vollständig, umschlossen sind, und/oder **dadurch gekennzeichnet, dass** das Kunststoffmaterial, das die Verstärkungselemente einbettet, auf der im Betrieb von dem Fluidgegendruck nicht belasteten Seite der Förderelemente wenigstens bereichsweise andere Eigenschaften aufweist als auf der mit dem Fluidgegendruck belasteten Seite der Förderelemente, insbesondere auf der von dem Fluidgegendruck nicht belasteten Seite stärker vernetzt oder geschrumpft ist oder dort auf der Oberfläche eine auf dem Förderelement (82) geschrumpfte Auflage (86) in Form von einer oder mehreren Folien, Beschichtungen oder Fasern trägt, und/oder **dadurch gekennzeichnet, dass** die Verstärkungselemente im zweiten, expandierten Zustand des Rotors ohne einen Fluidgegendruck in einem solchen Maß gestreckt vorliegen, dass sie sich beim Übergang zu einem dritten Zustand, der den Betriebszustand mit einem Fluidgegendruck darstellt, um weniger als 5 %, insbesondere weniger als 1 %, verlängern, wobei die Verlängerung insbesondere am Abstand der beiden Enden eines Verstärkungselementes voneinander bemessen ist.

13. Rotor nach Anspruch 1 oder einem der folgenden, **dadurch gekennzeichnet, dass** in einem zweiten, expandierten Zustand des Rotors und/oder einem dritten Betriebszustand mit Fluidgegendruck wenigstens ein Anteil von 10 %, weiter insbesondere mindestens 30 %, der Verstärkungselemente in wenigstens einem Bereich eines Förderelementes, in dem dieses gekrümmt ist, gestreckt und gerade verlaufen.

14. Rotor nach Anspruch 13, **dadurch gekennzeichnet, dass** in einem Bereich eines Förderelementes, in dem dieses gekrümmt ist, wenigstens zwei Anteile von Verstärkungselementen gerade und gestreckt verlaufen, wobei die Richtungen, in denen die Verstärkungselemente der Anteile verlaufen, bei dem jeweiligen Anteil parallel, jedoch bei den verschiedenen Anteilen verschieden sind.

15. Rotor nach Anspruch 1 oder einem der folgenden, **dadurch gekennzeichnet, dass** die Länge der Verstärkungselemente bei wenigstens 30 %, insbesondere wenigstens 50 %, der Elemente größer ist als die durchschnittliche Dicke der Förderelemente, insbesondere wenigstens zweimal so lang, weiter insbesondere wenigstens 5-mal oder 10-mal so lang, und/oder **dadurch gekennzeichnet, dass** Verstärkungselemente, insbesondere Fasern, in den sie einbettenden Kunststoff in einem Spritzgussverfahren eingebracht sind und während der Zeit, in der sich der Rotor in der Spritzgussform befindet, einen abschnittsweise gekrümmten Verlauf entlang der Strömung des Kunststoffs in die Spritzgussform aufweisen.

## Claims

1. A rotor for a compressible fluid pump, in particular a blood pump which can be introduced through a blood vessel into a patient's body, which rotor has one or more impeller elements (15) and can be radially compressed and expanded between a first, compressed state and a second, radially expanded state, and consists at least in part of a plastic reinforced by strand-like reinforcement elements, that is fibers (10, 11, 13, 18, 19, 55, 56, 62, 63), and is intended to rotate about an axis of rotation (14), wherein the rotor is tensioned in the first, compressed state and is free from external stresses in the second, expanded state, and wherein a third state exists, which the rotor (42) assumes in the operating state under load, **characterized in that** the reinforcing elements are enclosed by the plastic, from which the rotor is mostly made, at least on a portion of 90% of their surface,wherein the fibers in the rotor in the third state run stretched at least in sections, wherein in the second, expanded state of the rotor at least a portion of the reinforcing elements already run stretched and straight in at least one region of the impeller element in which it is curved.

2. The rotor as claimed in claim 1, **characterized in that** the reinforcement elements, in particular fibers (10, 11, 13, 18, 19, 55, 56, 62, 63), in the third state (operating state) of the rotor run in a stretched manner at least in part in regions of the rotor (42) in which this experiences an elongation stress and run substantially in the direction of the elongation stress.

3. The rotor as claimed in claim 1 or 2, **characterized in that** more than half of the reinforcement elements, in particular fibers (10, 11, 13, 18, 19, 55, 56, 62, 63), are arranged in regions of the rotor and/or the impeller elements in which this/these experiences/experience elongation stresses in the operating state.

4. The rotor as claimed in claim 1, 2 or 3, **characterized in that**, in the case of the rotor (42), there are substantially no differences between the second, expanded state and the third, operating state in respect of the outer form.

5. The rotor according to any one of the claims 1 to 4, **characterized in that** in the operating state at least part of the reinforcing elements, in particular fibers, in particular the majority of the reinforcing elements/fibers (10, 11, 13, 18, 19, 55, 56, 62, 63), run straight in sections.

6. The rotor according to any one of claims 1 to 4, **characterized in that** in the operating state at least a part of the reinforcing elements, in particular fibers (10, 11, 13, 18, 19, 55, 56, 62, 63), in particular the majority of the reinforcing elements/fibers, run along the longitudinal direction of the respective impeller element (15) with a smaller curvature than the neutral fiber (zero line) and/or neutral surface of the respective impeller element in the material mechanics sense.

7. The rotor as claimed in claim 1 or one of the following claims, **characterized in that** the plastic of the rotor (42) has a Shore hardness < 100 D, in particular < 80 D.

8. The rotor according to claim 1 or one of the following, **characterized in that** a first portion of more than 30%, in particular more than 50%, of the reinforcing elements/fibers (10, 11, 13, 18, 19, 55, 56, 62, 63) in the expanded state of the rotor (42) extends substantially stretched from a section (10a, 11a, 13a) closest to the rotor axis (14) to a second section (10b, 11b, 13b) further away from the axis of rotation (14).

9. The rotor according to claim 1 or one of the following, **characterized in that** a first portion of the reinforcing elements/fibers (10, 11, 13, 18, 19, 55, 56, 62, 63), which is more than 30% of the reinforcing elements/fibers, in particular more than 50%, of the reinforcing elements/fibers, has a length which corresponds to at least 30%, in particular at least 50%, of the maximum height of the impeller elements (15), measured in the radial direction of the rotor (42) in the expanded state.

10. The rotor according to claim 8 or 9, **characterized in that** the first portion of the reinforcing elements/fibers (10, 11, 13, 18, 19, 55, 56, 62, 63) is substantially perpendicular to the axis of rotation (14).

11. The rotor according to claim 1 or one of the following, **characterized in that** the diameter of the reinforcing elements/fibers (10, 11, 13, 18, 19, 55, 56, 62, 63), in particular of the first portion of the reinforcing elements/fibers (10, 11, 13, 18, 19, 55, 56, 62, 63), is smaller than 40 micrometers, and/or **characterized in that** the surface of the reinforcing elements/fibers (10, 11, 13, 18, 19, 55, 56, 62, 63) is provided with a bonding agent, and/or **characterized in that** a portion of reinforcing elements/fibers in the expanded state of the rotor extends transversely to the reinforcing elements/fibers of the first part and, in particular, encloses angles of at least 30° on average with this first part, and/or **characterized in that** the reinforcing elements/fibers are at least partially in the form of fabric sections having fibers running longitudinally and transversely, and/or**characterized in that** the reinforcing elements are in the form of film strips, the length of which is at least three times, in particular at least five times, further in particular at least ten times as great as their width.

12. The rotor according to claim 1 or any one of the following, **characterized in that** the reinforcing elements are enclosed by the plastic, from which the rotor is mostly made, at least on a portion of 99%, of their surface, more particularly completely, and/or **characterized in that** the plastic material, which embeds the reinforcing elements has different properties, at least in some areas, on the side of the conveying elements not subjected to the fluid counter pressure during operation than on the side of the conveying elements subjected to fluid counter pressure, in particular is more strongly cross-linked or shrunk on the side not subjected to fluid counter pressure, or bears on its surface an overlay (86) in the form of one or more films, coatings or fibers which is shrunk onto the delivery element (82),and/or **characterized in that** the reinforcement elements in the second, expanded state of the rotor, without a fluid counter pressure, are present in a form stretched to such an extent that when transitioning to a third state, which constitutes the operating state with a fluid counter pressure, they are lengthened by less than 5%, in particular less than 1%, wherein the lengthening is measured in particular on the basis of the distance between the two ends of a reinforcement element.

13. The rotor according to claim 1 or one of the following, **characterized in that** in a second, expanded state of the rotor and/or a third operating state with fluid counter pressure at least 10%, more particularly at least 30% of the reinforcement elements, run in a stretched and straight manner in at least one region of an impeller element in which said impeller element is curved.

14. The rotor according to claim 13, **characterized in that** in a region of a conveying element in which the latter is curved, at least two portions of reinforcing elements run straight and stretched, wherein the directions in which the reinforcing elements of the portions run are parallel for the respective portion but different for the different portions.

15. The rotor according to claim 1 or one of the following, **characterized in that** the length of the reinforcement elements in the case of at least 30%, in particular at least 50% of the elements is greater than the average thickness of the impeller elements, in particular at least twice as long, more particularly at least five times or ten times as long., and/or **characterized in that** reinforcing elements, in particular fibers, are introduced into the plastic embedding them in an injection molding process and, during the time in which the rotor is in the injection mold, have a sectionally curved profile along the flow of the plastic into the injection mold.

## Revendications

1. Rotor pour pompe à fluide pouvant être comprimée, en particulier pompe sanguine pouvant être implantée dans un corps de patient en passant par un vaisseau sanguin, qui présente un ou plusieurs élément(s) de transport (15) et qui peut être comprimé et dilaté radialement entre un premier état comprimé et un deuxième état radialement dilaté, et qui est constitué au moins partiellement d'un matériau plastique renforcé par des éléments de renfort filiformes, à savoir des fibres (10, 11, 13, 18, 19, 55, 56, 62, 63), et qui est conçu pour tourner autour d'un axe de rotation (14), dans lequel le rotor est soumis à une tension dans le premier état comprimé et est exempt de tensions extérieures dans le deuxième état dilaté, et dans lequel il existe un troisième état où le rotor (42) en fonctionnement sous charge, **caractérisé en ce que** les fibres sont enserrées sur au moins 90 % de leur surface dans la matière plastique dont est principalement constitué le rotor, dans lequel, dans le troisième état, les fibres au sein du rotor s'étendent en étant étirées au moins par endroits, dans lequel, dans le deuxième état dilaté du rotor, au moins une part des éléments de renfort s'étendent déjà en étant étirés et droits dans au moins une région de l'élément de transport où celui-ci est incurvé.

2. Rotor selon la revendication 1, **caractérisé en ce que**, dans le troisième état (état de fonctionnement) du rotor, les éléments de renfort, en particulier des fibres (10, 11, 13, 18, 19, 55, 56, 62, 63), s'étendent en étant étiré(e)s au moins par endroits dans des régions du rotor (42) au sein desquelles celui-ci présente une tension d'allongement et s'étendent essentiellement dans la direction de la tension d'allongement.

3. Rotor selon la revendication 1 ou 2, **caractérisé en ce que** plus de la moitié des éléments de renfort, en particulier des fibres (10, 11, 13, 18, 19, 55, 56, 62, 63), sont agencé(e)s dans des régions du rotor et/ou des éléments de transport au sein desquelles celui-ci/ceux-ci présente/présentent des tensions d'allongement dans l'état de fonctionnement.

4. Rotor selon l'une quelconque des revendications 1, 2 ou 3, **caractérisé en ce que** le rotor (42) ne présente essentiellement aucune différence de forme extérieure entre le deuxième état dilaté et le troisième état de fonctionnement.

5. Rotor selon l'une quelconque des revendications 1 à 4, **caractérisé en ce que**, dans l'état de fonctionnement, au moins une partie des éléments de renfort, en particulier des fibres, en particulier la majorité des éléments de renfort/des fibres (10, 11, 13, 18, 19, 55, 56, 62, 63), s'étendent de manière rectiligne par endroits.

6. Rotor selon l'une quelconque des revendications 1 à 4, **caractérisé en ce que**, dans l'état de fonctionnement, au moins une partie des éléments de renfort, en particulier des fibres (10, 11, 13, 18, 19, 55, 56, 62, 63), en particulier la majorité des éléments de renfort/des fibres s'étendent le long de la direction longitudinale de l'élément de transport (15) respectif avec une courbure inférieure à celle de la fibre neutre (ligne zéro) et/ou à celle de la surface neutre de l'élément de transport respectif au regard de la résistance des matériaux.

7. Rotor selon la revendication 1 ou l'une quelconque des revendications suivantes, **caractérisé en ce que** la matière plastique du rotor (42) présente une dureté Shore < 100 D, en particulier < 80 D.

8. Rotor selon la revendication 1 ou l'une quelconque des revendications ci-dessous, **caractérisé en ce qu'**une première part de plus de 30 %, en particulier de plus de 50 %, des éléments de renfort/des fibres (10, 11, 13, 18, 19, 55, 56, 62, 63) à l'état dilaté du rotor (42) s'étendent essentiellement en étant étiré(e)s à partir d'une section (10a, 11a, 13a) située au plus près de l'axe de rotation (14) jusqu'à une seconde section (10b, 11b, 13b) située au plus loin de l'axe de rotation (14).

9. Rotor selon la revendication 1 ou l'une quelconque des revendications ci-dessous, **caractérisé en ce qu'**une première part des éléments de renfort/des fibres (10, 11, 13, 18, 19, 55, 56, 62, 63), représentant plus de 30 % des éléments de renfort/des fibres, en particulier plus de 50 % des éléments de renfort/des fibres, présentent une longueur qui correspond à au moins 30 %, en particulier au moins 50 %, de la hauteur maximale des éléments de transport (15), mesurée dans la direction radiale du rotor (42) à l'état dilaté.

10. Rotor selon la revendication 8 ou 9, **caractérisé en ce que** la première part des éléments de renfort/des fibres (10, 11, 13, 18, 19, 55, 56, 62, 63) s'étendent de manière essentiellement perpendiculaire à l'axe de rotation (14).

11. Rotor selon la revendication 1 ou l'une quelconque des revendications ci-dessous, **caractérisé en ce que** le diamètre des éléments de renfort/des fibres (10, 11, 13, 18, 19, 55, 56, 62, 63), en particulier de la première part des éléments de renfort/des fibres (10, 11, 13, 18, 19, 55, 56, 62, 63), est inférieur à 40 micromètres, et/ou **caractérisé en ce que** la surface des éléments de renfort/des fibres (10, 11, 13, 18, 19, 55, 56, 62, 63) est munie d'un promoteur d'adhérence, et/ou **caractérisé en ce qu'**une part des éléments de renfort/des fibres s'étendent, à l'état dilaté du rotor, transversalement par rapport aux éléments de renfort/des fibres de la première partie et forme en particulier avec lesdit(e)s éléments de renfort/fibres un angle d'au moins 30° en moyenne, et/ou caractérisé en que les éléments de renfort/les fibres sont au moins partiellement présent(e)s sous forme de sections de tissu comprenant des fibres s'étendant longitudinalement et transversalement, et/ou **caractérisé en ce que** les éléments de renfort sont présents sous forme de bandes de feuille dont la longueur est au moins trois fois, en particulier au moins cinq fois, et plus particulièrement au moins dix fois supérieure à leur largeur.

12. Rotor selon la revendication 1 ou l'une quelconques des revendications ci-dessous, **caractérisé en ce que** les éléments de renfort sont enserrés sur au moins 99 % de leur surface, et plus particulièrement totalement, dans la matière plastique dont le rotor est principalement constitué, et/ou **caractérisé en ce que** la matière plastique qui enrobe les éléments de renfort présente, au moins dans certaines régions, du côté des éléments de transport qui n'est pas soumis à la contre-pression de fluide pendant le fonctionnement, des propriétés différentes de celles présentées du côté des éléments de transport qui est soumis à la contre-pression de fluide, en particulier présente une réticulation ou un retrait plus important du côté qui n'est pas soumis à la contre-pression de fluide, ou présente en surface une couche rétractée (86) sur l'élément de transport (82) sous la forme d'un(e) ou de plusieurs film(s), revêtement(s) ou fibre(s), et/ou **caractérisé en ce que** les éléments de renfort, dans le deuxième état dilaté du rotor sans une contre-pression de fluide, sont présents en étant étirés dans une mesure telle qu'ils s'allongent de moins de 5 %, et en particulier de moins de 1 %, lors du passage à un troisième état représentant l'état de fonctionnement avec une contre-pression de fluide, dans lequel l'allongement se mesure en particulier au niveau de l'espacement entre les deux extrémités d'un élément de renfort.

13. Rotor selon la revendication 1 ou l'une quelconque des revendications ci-dessous, **caractérisé en ce que**, dans un deuxième état dilaté du rotor et/ou un troisième état de fonctionnement avec contre-pression de fluide, au moins une part de 10 %, en particulier au moins de 30 %, des éléments de renfort s'étendent en étant étirés et rectilignes dans au moins une région d'un élément de transport au sein de laquelle celui-ci est incurvé.

14. Rotor selon la revendication 13, **caractérisé en ce que**, dans une région d'un élément de transport au sein de laquelle celui-ci est incurvé, au moins deux parts d'éléments de renfort s'étendent en étant rectilignes et étirés, dans lequel les directions dans lesquelles les éléments de renfort des parts s'étendent sont parallèles pour chaque part, mais sont différentes pour les différentes parts.

15. Rotor selon la revendication 1 ou l'une quelconque des revendications ci-dessous, **caractérisé en ce que** la longueur des éléments de renfort pour au moins 30 %, en particulier pour au moins 50 %, des éléments est supérieure à l'épaisseur moyenne des éléments de transport, en particulier au moins deux fois supérieure, plus particulièrement au moins 5 ou 10 fois supérieure, et/ou **caractérisé en ce que** les éléments de renfort, en particulier les fibres, sont introduit(e)s dans la matière plastique les enrobant au cours d'un procédé de moulage par injection et, pendant la durée où le rotor se trouve dans le moule d'injection, présentent un tracé incurvé par endroits le long de l'écoulement de la matière plastique dans le moule d'injection.
